# EUROPEAN PATENT SPECIFICATION

(11) **EP 2 817 024 B1**
(45) Date of publication and mention of the grant of the patent: **10.07.2019**
(21) Application number: 13708044.6
(22) Date of filing: 22.02.2013
(51) Int. Cl.: A61K 38/16, A61K 38/17, A61K 45/06, A61K 9/51, A61K 33/00, A61P 35/00, A61P 37/00, A61P 3/00

(54) **NANOSTRUCTURES FOR TREATING CANCERS**
NANOSTRUKTUREN ZUR BEHANDLUNG VON KREBS
NANOSTRUCTURES PERMETTANT DE TRAITER DES CANCERS

(30) Priority: 22.02.2012 US 201261601706 P
(43) Date of publication of application: 31.12.2014
(60) Divisional application: 19175659.2
(73) Proprietor: Northwestern University, Evanston, IL 60208 (US)
(72) Inventor: THAXTON, C., Shad, Chicago, IL 60614 (US); DAMIANO, Marina, G., Chicago, IL 60611 (US); ZHANG, Heng, Chicago, IL 60615 (US); MCMAHON, Kaylin, M., Chicago, IL 60614 (US); YANG, Shuo, Lisle, IL 60532 (US); GORDON, Leo, I., Winnetka, IL 60093 (US); SINGH, Amareshwar, T.k., Buffalo Grove, IL 60089 (US)
(74) Representative: Hoffmann Eitle
(86) International application number: PCT/US2013/027431
(87) International publication number: WO 2013/126776

(56) References cited:
- WO-A2-2007/089607
- WO-A2-2011/091065
- BOUDREAULT PIERRE-LUC ET AL: "Nanoscale tools to selectively destroy cancer cells.", CHEMICAL COMMUNICATIONS (CAMBRIDGE, ENGLAND) 14 MAY 2008, no. 18, 14 May 2008 (2008-05-14), pages 2118-2120, XP002696352, ISSN: 1359-7345
- GONCALVES R P ET AL: "Uptake of high density lipoprotein (HDL) cholesteryl esters by human acute leukemia cells", LEUKEMIA RESEARCH, NEW YORK,NY, US, vol. 29, no. 8, 1 August 2005 (2005-08-01) , pages 955-959, XP027722050, ISSN: 0145-2126 [retrieved on 2005-08-01]
- M. F. MULAS ET AL: "Cholesterol esters as growth regulators of lymphocytic leukaemia cells", CELL PROLIFERATION, vol. 44, no. 4, 1 August 2011 (2011-08-01) , pages 360-371, XP055061483, ISSN: 0960-7722, DOI: 10.1111/j.1365-2184.2011.00758.x
- DESSI ET AL: "Role of cholesterol synthesis and esterification in the growth of CEM and MOLT4 lymphoblastic cells.", BIOCHEMICAL JOURNAL, vol. 321, no. 3, 1 February 1997 (1997-02-01), pages 603-608, XP055061485, ISSN: 0264-6021
- ROTHBLAT G H ET AL: "Cell cholesterol efflux: integration of old and new observations provides new insights.", JOURNAL OF LIPID RESEARCH MAY 1999, vol. 40, no. 5, May 1999 (1999-05), pages 781-796, XP002696353, ISSN: 0022-2275
- ACTON S ET AL: "Identification of scavenger receptor SR-BI as a high density lipoprotein receptor", SCIENCE, AMERICAN ASSOCIATION FOR THE ADVANCEMENT OF SCIENCE, WASHINGTON, DC; US, vol. 271, 26 January 1996 (1996-01-26), pages 518-520, XP002090829, ISSN: 0036-8075, DOI: 10.1126/SCIENCE.271.5248.518
- Y. JI ET AL: "Scavenger Receptor BI Promotes High Density Lipoprotein-mediated Cellular Cholesterol Efflux", JOURNAL OF BIOLOGICAL CHEMISTRY, vol. 272, no. 34, 22 August 1997 (1997-08-22), pages 20982-20985, XP055061487, ISSN: 0021-9258, DOI: 10.1074/jbc.272.34.20982
- CARLOS G. LEON ET AL: "Alterations in cholesterol regulation contribute to the production of intratumoral androgens during progression to castration-resistant prostate cancer in a mouse xenograft model", THE PROSTATE, 1 January 2009 (2009-01-01), pages n/a-n/a, XP055061488, ISSN: 0270-4137, DOI: 10.1002/pros.21072
- SILVIA R. GRAZIANI ET AL: "Uptake of a Cholesterol-Rich Emulsion by Breast Cancer", GYNECOLOGIC ONCOLOGY, vol. 85, no. 3, 1 June 2002 (2002-06-01), pages 493-497, XP055061490, ISSN: 0090-8258, DOI: 10.1006/gyno.2002.6654
- THAXTON C SHAD ET AL: "Templated spherical high density lipoprotein nanoparticles", JOURNAL OF THE AMERICAN CHEMICAL SOCIETY, ACS PUBLICATIONS, US, vol. 131, no. 4, 4 February 2009 (2009-02-04), pages 1384-1385, XP002587918, ISSN: 0002-7863, DOI: 10.1021/JA808856Z [retrieved on 2009-01-09]

## Description

### FIELD OF INVENTION

The present invention generally relates to nanostructures and compositions for treating cancers and other conditions.

### BACKGROUND

Nanostructures, including liposome nanostructures, are currently being used in applications such as drug delivery, gene delivery, and diagnostics. A variety of methods have been used to fabricate such nanostructures; for example, liposome nanostructures have been formed by techniques including lipoprotein/conjugate synthesis and sonicating mixtures of amphipathic liposome components. However, some such methods often lead to structures having relatively large sizes, large size distributions, and/or short term stability. Accordingly, a need exists for nanostructures having smaller sizes, controlled size distributions, and/or long term stability, and methods for making such nanostructures, while being able to control functionality and tailorability of the nanostructures. The use of such nanostructures for treating conditions such as cancer would also be beneficial.

### SUMMARY OF THE INVENTION

The present invention generally relates to nanostructures and compositions for treating cancers. In some cases, the nanostructure may be used to treat cancers by controlling cholesterol metabolism in cells. The nanostructures and compositions may be used, for example, to control cholesterol influx and efflux in cancer cells. The nanostructures are mimics of natural HDL as described in more detail below. The subject matter of this application involves, in some cases, interrelated products, alternative solutions to a particular problem, and/or a plurality of different uses of structures and compositions, as will be described in more details below.

The present invention is defined in the appended claims, while embodiments not falling under these claims are for reference purpose only.

In one set of embodiments, a series of methods are provided.

In one set of embodiments, a method for killing cancer cells having scavenger receptor type B-I (SR-B1) is provided. The method involves contacting the cancer cells having SR-B1 with a synthetic nanostructure in an amount effective to kill the cancer cells.

In one set of embodiments, a method for killing B-cell lymphoma cells is provided. The method involves contacting the B-cell lymphoma cells with a synthetic nanostructure in an amount effective to kill the B-cell lymphoma cells.

In one set of embodiments, a method for treating non-Hodgkin lymphoma in a subject is provided. The method involves administering to the subject a synthetic nanostructure in an amount effective to treat the non-Hodgkin lymphoma.

In one set of embodiments, a method for treating cancer in a subject is provided. The method involves administering to the subject a composition that controls cholesterol influx and efflux in cancer cells of the subject to treat the cancer.

In one set of embodiments, a method of diagnosing, preventing, treating, or managing a disease or bodily condition is provide. The method involves administering to a subject a therapeutically-effective amount of a composition comprising a synthetic structure comprising a nanostructure core and a shell surrounding and attached to the nanostructure core, allowing the synthetic nanostructure to bind with a cell surface receptor that binds a natural lipoprotein, and blocking or reducing the amount of binding between the cell surface receptor and the natural lipoprotein.

In some embodiments, the natural lipoprotein is HDL, IDL, LDL, or VLDL. The method may involve, for example, increasing or decreasing cholesterol influx or efflux in a cell comprising the cell surface receptor.

In one set of embodiments, a method for diagnosing, preventing, treating, or managing a disease or bodily condition associated with abnormal lipid levels is provided. The method may involve administering to a subject a therapeutically-effective amount of a composition comprising a synthetic structure comprising a nanostructure core and a shell surrounding and attached to the nanostructure core, and altering cellular cholesterol flux in the subject using the synthetic structure.

In one set of embodiments, a method involves administering to a biological matrix a therapeutically-effective amount of a composition comprising a synthetic structure comprising a nanostructure core and a shell surrounding and attached to the nanostructure core, and allowing the synthetic nanostructure to sequester or exchange lipids or proteins with other natural lipoproteins in the biological matrix. The synthetic nanostructure may be a mimic of the natural lipoprotein. In some embodiments, the method involves binding the synthetic nanostructure to a cell surface receptor that can bind with a natural lipoprotein.

In any one of the methods described above, the method may involve binding the structure, or a component of the structure, to one or more cell surface receptors that regulate cholesterol transport. The cell surface receptor may be, for example, SR-B1, ABCA1 and/or ABCG1.

In any one of the methods described above, the disease or bodily condition associated with abnormal lipid levels may involves inflammation or regulating the immune system.

In any one of the methods described above, the composition may comprise a plurality of synthetic nanostructures. In some embodiments, the cancer is non-Hodgkin lymphoma. In some cases, the cancer is characterized by B-cell lymphoma cells, or wherein the cancer cells are B-cell lymphoma cells. In some embodiments, the cancer is leukemia. In other embodiments, the cancer is melanoma. In certain embodiments, the cancer is characterized by cells having SR-B1. In some embodiments, the cancer is characterized by cells having ABCA1 and/or ABCG1, or wherein the cancer cells have ABCA1 and/or ABCG1.

In any one of the methods described above, a method may involve controlling the growth of the cancer cells. In some embodiments, a method involves killing the cancer cells. In some cases, killing takes place by apoptosis. In some embodiments, a method involves controlling cholesterol metabolism in the cancer cells. Controlling cholesterol metabolism may involve, for example, increasing cholesterol efflux out of the cancer cells and/or decreasing cholesterol influx into the cancer cells. In some cases, a method involves modulating SR-B1 binding. In some instances, a method involves substantially inhibiting SR-B1.

In any one of the methods described above, a method may involve using the synthetic nanostructure to sequester cholesterol in the cancer cells. The synthetic nanostructure may be used to sequester at least 5, at least 10, at least 20, or at least 50 of cholesterol molecules in the cancer cells. The cholesterol may be, for example, esterified cholesterol or free cholesterol.

In any one of the methods described above, the synthetic nanostructure may be a biomimic of mature, spherical high-density lipoprotein, e.g., with respect to size, shape, and/or surface chemistry. The synthetic nanostructure may be adapted to sequester cholesterol. In some embodiments, the synthetic nanostructure comprises a nanostructure core and a shell. In some cases, a nanostructure core includes an inorganic material, such as a metal (e.g., gold).

In any one of the methods described above, the synthetic nanostructure core may have a largest cross-sectional dimension of less than or equal to about 50 nm, or less than or equal to about 35 nm, or less than or equal to about 30 nm.

In any one of the methods described above, the synthetic nanostructure may comprise a shell comprising a lipid layer surrounding and attached to a nanostructure core. In some cases, the lipid layer is a lipid bilayer. In some embodiments, at least a portion of the lipid bilayer is covalently bound to the core. In other embodiments, at least a portion of the lipid bilayer is physisorbed to the core. In some cases, the lipid bilayer comprises a phospholipid. For example, the lipid bilayer may comprise 50-200 phospholipids. In some cases, the shell comprises a lipoprotein structure. In some embodiments, the shell comprises an apolipoprotein. The apolipoprotein may be, for example, apolipoprotein A-I, apolipoprotein A-II, or apolipoprotein E. In some embodiments, the synthetic nanostructure includes 1-6 apolipoproteins. In certain embodiments, the synthetic nanostructure comprises a shell having an inner surface and an outer surface, and a protein is associated with at least the outer surface of the shell.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present disclosure will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
FIG. 1A shows an example of a nanostructure that can be used to treat a disease or condition described herein according to one set of embodiments;
FIG.1B shows a method of fabricating nanostructures according to one set of embodiments;
FIG. 2A shows relative SR-B1 expression by gene expression profiling in lymphoma patient samples as compared to naive and memory B cells obtained from healthy donors according to one set of embodiments;
FIG. 2B is an image of a Western blot showing the expression of high-density lipoprotein (HDL)-specific receptors, ABCA1, ABCG1, and SR-B1 in lymphoma cell lines according to one set of embodiments;
FIG. 2C shows a Western blot demonstrating the expression of SR-B1 in lymphoma and normal lymphocytes (Normal HL). Numbers represent the ratio of SR-B1 receptor expression to GAPDH. All ratios were normalized to the same ratio measured for SR-B1 expression in HepG2 cells according to one set of embodiments;
FIG. 2D shows expression of HDL receptor SR-B1 in all cultured cancer cell lines except for Jurkat according to one set of embodiments;
FIG. 2E shows SR-B1 expression in hepatocytes and macrophages differentiated from human CD14+ cells. HepG2 and Jurkat cells were included as SR-B1-positive and -negative controls, respectively, according to one set of embodiments;
FIGS. 3A and 3B show the effect of hHDL and HDL-NP on lymphoma cells MTS assay (72 h) for the effect of (A) hHDL and (B) HDL-NP according to one set of embodiments. The absorbance values measures in the control (untreated cells) was set to 100% for all MTS assays;
FIGs. 3C and 3D show the effect of hHDL and HDL-NP on primary cells according to one set of embodiments. MTS assays show that the hHDL and HDL-NPs do not significantly reduce the viability of (A) primary hepatocytes or (B) primary macrophages at 24 and 48 h post-treatment. At 72 h post-treatment, hHDL significantly increase the viability of both hepatocytes and macrophages compared to the control;
FIG.4 shows the effects of free components of synthetic nanostructures described herein (e.g., HDL-NP) on lymphoma cells according to one set of embodiments;
FIG. 5A shows results of an ³H thymidine incorporation assay to measure cell proliferation in the presence of Ac-LDL and HDL-NPs in lymphoma cell lines according to one set of embodiments;
FIG. 5B shows that apoptosis (72 h) of HDL-NP-treated lymphoma cells is dose dependent according to one set of embodiments;
FIG. 5C shows a colorimetric assay for activated caspase 3 activity according to one set of embodiments;
FIGs. 5D-5F show Western blots for PARP and caspase-3 according to one set of embodiments: 28 h Western blot for (FIG. 5D) HDL-NP-induced cleavage of PARP, (FIG. 5E) levels of caspase 3 in Ramos cells, and (FIG. 5F) 24 h Western blot for HDL-NP-induced cleavage of PARP in SUDHL-4 cells;
FIG. 6A shows apoptosis in primary hepatocytes and macrophages after 10 nM hHDL and HDL-NP treatment according to one set of embodiments;
FIG. 6B shows apoptosis in HDL-NP-treated normal human lymphocytes (normal lymph) and SUDHL-4 cells expressed as fold increase compared with control (48 h) according to one set of embodiments. (Inset) Apoptosis in normal human lymphocytes at 2 or 5 d after treatment with HDL-NPs;
FIG. 7A shows HDL-NP uptake in lymphoma cells by ICP-MS data measuring Au content according to one set of embodiments;
FIG. 7B shows HDL-NP uptake in lymphoma cells by ICP-MS-based competition experiment between HDL-NP and hHDL according to one set of embodiments;
FIG. 7C shows transmission electron micrographs of HDL-NP in SUDHL-4 cells after HDL-NP treatment (24 h) according to one set of embodiments;
FIG. 8A shows MTS assay results of Ac-LDL (micrograms per milliliter) rescue in SR-B1⁻ and SR-B1⁺ lymphoma cell lines according to one set of embodiments;
FIG. 8B shows apoptosis in lymphoma cells lines after rescue with Ac-LDL;
FIGS. 8C and 8D show apoptosis in treated Ramos cells and SUDHL-4 cells, respectively, according to one set of embodiments;
FIGS. 8E and 8F show representative Annexin V/PI flow cytometry histograms of apoptosis in Ramos and SUDHL-4, respectively, according to one set of embodiments;
FIG. 9A shows the percent cholesterol efflux from lymphoma cells to HDL-NPs compared with hHDL according to one set of embodiments;
FIG. 9B shows cholesterol efflux from human hepatocytes and macrophages to hHDL and HDL-NPs according to one set of embodiments;
FIG. 9C shows cholesterol influx to lymphoma cells by HDL-NPs and hHDL according to one set of embodiments;
FIG. 9D shows cholesterol influx to human hepatocytes and macrophages from hHDL and HDL-NP according to one set of embodiments;
FIG. 9E shows cholesterol efflux from SUDHL-4 cells to hHDL and HDL-NP alone and after treatment with 10 µM BLT-1 according to one set of embodiments;
FIG. 9F shows cholesterol influx from hHDL and HDL-NP to SUDHL-4 cells alone and after treatment with 10 µM BLT-1 according to one set of embodiments;
FIGS. 10A and 10B show the effect of HDL-NP on tumor volume and SR-B1 expression levels in a xenograft model for Ramos and Jurkat tumors, respectively, according to one set of embodiments;
FIG. 11A shows SR-B1 expression levels in Ramos and Jurkat tumors of four representative mice harvested at day 11 according to one set of embodiments;
FIGs. 11B-11E show H&E staining of tumor samples according to one set of embodiments. (FIG. 11B) Jurkat tumor specimen surrounded by adipose tissue; (FIG. 11C) Higher magnification image (5x) also reveals adipocyte contamination within the Jurkat tumor; (FIG. 11D and 11E) 10x magnification, adipocyte contamination is more prevalent in (FIG. 11D) Jurkat tumor than (FIG. 11E) Ramos tumor;
FIG. 12A shows the effects of synthetic nanostructures described herein (e.g., HDL-NP) on human umbilical vein endothelial cells (HUVEC) according to one set of embodiments;
FIG. 12B shows the effects of synthetic nanostructures described herein (e.g., HDL-NP) on HepG2 cells (human liver cancer cells) according to one set of embodiments;
FIG. 12C shows the effects of synthetic nanostructures described herein (e.g., HDL-NP) on PC3 cells (human prostate cancer cells) according to one set of embodiments;
FIG. 12D shows the effects of synthetic nanostructures described herein (e.g., HDL-NP) on LnCaP cells (human prostate cancer cells) according to one set of embodiments;
FIG. 12E shows the effects of synthetic nanostructures described herein (e.g., HDL-NP) on HMLE-GFP cells (human breast epithelial cells) according to one set of embodiments;
FIG. 12F shows the effects of synthetic nanostructures described herein (e.g., HDL-NP) on HMLE-Twist cells (human breast epithelial cells) according to one set of embodiments;
FIG. 12G shows the effects of synthetic nanostructures described herein (e.g., HDL-NP) on MDA-MB-231 cells (human breast cancer cells) according to one set of embodiments;
FIG. 12H shows the effects of synthetic nanostructures described herein (e.g., HDL-NP) on C8161 cells (human melanoma cells) according to one set of embodiments;
FIG. 12I shows the effects of synthetic nanostructures described herein (e.g., HDL-NP) on A375 cells (human melanoma cells) according to one set of embodiments;
FIG. 12J shows the effects of synthetic nanostructures described herein (e.g., HDL-NP) on Ramos cells (human B-cell lymphoma cells) according to one set of embodiments; and
FIG. 13 is a UV-vis spectrum of a synthetic nanostructure (HDL-NP) according to one set of embodiments.

### DETAILED DESCRIPTION

Nanostructures, compositions and methods for treating cancers and other conditions are provided. In some cases, the nanostructures and/or compositions may be used to treat cancers or other diseases or conditions at least in part by controlling cholesterol metabolism in cells. The nanostructures and compositions may be used, for example, to control cholesterol influx and/or efflux in cells. In some cases, the nanostructures or compositions may be used to kill the cells. Examples of cancer cells that may be affected by the nanostructures and compositions described herein include those having scavenger receptor type B-I (SR-B1), B-cell lymphoma cells, non-Hodgkin's lymphoma cells, melanoma cells, and/or others. In some embodiments, the nanostructures may be mimics of natural high-density lipoprotein (HDL).

Described herein are novel therapeutic approaches to target cancer cells (e.g., cells having the receptor SR-BI, lymphoma cells, non-Hodgkin's lymphoma cells, melanoma cells and/or others) using a synthetic nanostructure and/or compositions thereof. In some embodiments, the synthetic nanostructure may be a biomimic of mature, spherical HDL, e.g., in terms of the size, shape, surface chemistry and/or function of the structures. Control of such features may be accomplished at least in part by using a synthetic template for the formation of the nanostructures. For example, high-density lipoprotein synthetic nanoparticles (HDL-NP) may be formed by using a gold nanoparticle (Au-NP) (or other suitable entity or material) as a synthetic template to which other components (e.g., lipids, proteins, etc.) can be added.

In some embodiments, the synthetic nanostructures may have a substantially similar size, shape and/or surface chemistry to that of natural HDL, but may differ in at least one characteristic from that of natural HDL. The at least one characteristic may be, for example, the presence or absence of one or more components in the nanostructure, the positioning of one or more components in or on the nanostructure, the materials used to form the nanostructure, the makeup of the shell of the nanostructure, the makeup of the core of the nanostructure, and combinations thereof. For example, in some embodiments, the nanostructures can be made substantially free of cholesterol (e.g., in the core, and/or prior to administration of the nanostructures to a subject or sample) as the Au-NP or other suitable entity occupies the real-estate at the core. This configuration differs from that of natural HDLs, which have a core formed of cholesteryl esters and triglycerides. Furthermore, the nanostructures described herein may have certain characteristics and/or functions similar to that of natural HDL (e.g., cholesterol binding constant) but may have other characteristics and/or functions that differ from that of natural HDL (e.g., ability to deliver cholesterol to cells). The differences between the nanostructures described herein and natural HDLs may contribute to the effectiveness of the nanostructures in treating the cells, diseases and conditions described herein, as described in more detail below.

Cholesterol in cell membranes appears to be important for the survival and proliferation of malignant cells. As a therapy, and in direct contrast to natural HDL, the nanostructures described herein may induce time and dose-dependent apoptosis in cancer cells such as those having scavenger receptor type B-I (SR-B1), B-cell lymphoma cells, non-Hodgkin's lymphoma cells, melanoma cells and/or others. For example, data demonstrate that the therapeutic efficacy of the nanostructures may be derived by targeting the high affinity receptor for HDL, SR-B1, and/or by the ability of the nanostructure to manipulate cellular cholesterol flux. Taken together, targeted nanostructure receptor binding, which may be achieved through the surface chemistry of the nanostructures, and/or manipulation of cellular cholesterol flux, which may be achieved through features of the nanostructure such as the occupancy of the core, may uniquely cooperate to deliver significant potential therapeutic efficacy as an alternative to chemotherapy for cancers such as B-cell lymphoma, non-Hodgkin's lymphoma, melanoma or others. More generally, the structures and methods described herein may be applicable across a continuum of cells that are sensitive to cholesterol flux as a means of survival, and also in cells where cholesterol accumulation is pathologic.

Accordingly, in one set of embodiments, methods for treating cancer in a subject are provided. In one embodiment, a method may involve administering to the subject a composition that controls cholesterol influx and efflux in cancer cells of the subject to treat the cancer. In some cases, the cancer is identified by cells that require the maintenance of cholesterol in order to survive. In one embodiment, a method for treating cancer, wherein the cancer is defined by cancer cells expressing SR-B1, is provided. In one embodiment, a method of killing cancer cells having SR-B1 is provided. The method may involve contacting the cancer cells having SR-B1 with a synthetic nanostructure in an amount effective to kill the cancer cells. In other embodiments, a method of reducing the proliferation or viability of cancer cells is provided. The cancer may be defined, in some embodiments, by certain cancer cells expressing SR-B1. Non-limiting examples of such cells include Ramos cells, SUDHL4 cells, LY3 cells, melanoma cells (e.g., A375 and/or C8161 cell lines), and B-cell lymphoma cells.

In certain embodiments, the cancer cells to be treated express the receptors ABCA1 and/or ABCG1. In one embodiment, a method for killing B-cell lymphoma cells is provided. The method may involve contacting the B-cell lymphoma cells with a synthetic nanostructure in an amount effective to kill the B-cell lymphoma cells. In one embodiment, a method for treating non-Hodgkin lymphoma in a subject is provided. The method may involve administering to the subject a synthetic nanostructure in an amount effective to treat the non-Hodgkin lymphoma. In other embodiments, the nanostructures described herein may be used to treat leukemia, lymphoma, and/or melanoma. In certain embodiments, the nanostructures described herein can be used to treat diseases or conditions involving the process of pathologic cholesterol accumulation, especially diseases or conditions identified by cells having receptors in which the cellular expression of the receptors are naturally engaged by HDL and/or the nanostructures described herein. Other cancers or conditions may also benefit from aspects of the invention.

Surprisingly, the nanostructures described herein, which may be designed to mimic the size, shape, surface chemical composition, and/or cholesterol binding properties of natural, mature spherical HDL, were shown to kill cancer cells that express SR-B1, including B-cell lymphoma cells and/or cells from patients having non-Hodgkin's lymphoma. While it was thought that the nanostructures could be used to sequester cholesterol in cells (similar to the function of natural HDL), it was unexpected that the nanostructures would kill the cells, e.g., by apoptosis, especially when the nanostructures did not incorporate any drugs or chemotherapeutic agents to specifically treat the cancer cells. This is especially the case since natural HDL (which the nanostructure may be designed to mimic) has no killing effect in cell lines that express SR-B1, and that natural HDL actually leads to an increase in proliferation of cells that express this receptor. It was unexpected that the nanostructures described herein would have such a drastic and opposite effect of not only controlling the growth of cancer cells by decreasing the proliferation of cells expressing SR-B1 (including B-cell lymphoma cells and/or cells from patients having non-Hodgkin's lymphoma), but also killing the cells. It was also unexpected that cell death was brought about by apoptosis, as there are several possible mechanisms for cell death including autophagy and necrosis/cell lysis.

There are approximately 70,000 new cases of non-Hodgkin lymphoma (NHL) each year, and 90% are B-cell lymphomas. These cause an estimated 19,320 deaths per year. Current treatment, while more highly effective now than in the past, includes chemotherapy, radiation, small molecule inhibitors of signaling pathways, and immunotherapy; however, resistance develops and results in disease progression and death. Thus, new and more effective treatment strategies are needed. Recent evidence gathered in lymphoblasts and myeloblasts from patients with acute lymphocytic leukemia (ALL) and acute myeloid leukemia (AML) demonstrates enhanced uptake of cholesterol through high-density lipoprotein carriers, which may result in increased cell proliferation. In addition, enhanced esterification of cholesterol within leukemia and lymphoma cell lines is correlated with increased cellular proliferation and small molecule inhibition of cholesterol ester formation was shown to inhibit cell growth. Taken together, these data suggest that cholesterol and its metabolites are important for the survival and proliferation of B-cell NHL.

High-density lipoproteins are dynamic natural nanoparticles that are important because of the inverse correlation that exists between circulating HDL levels and the development of cardiovascular disease. One of the most important atheroprotective mechanisms of HDLs is hypothesized to be reverse cholesterol transport (RCT). RCT is a complex process of cholesterol efflux from lipid-laden macrophages found in developing atheromas to HDL particles, cholesterol transport in the peripheral circulation, and delivery of cholesterol to the liver for excretion in the feces. During the process of RCT, cholesterol uptake chemically and structurally matures HDLs from initial nascent, discoidal forms to more mature spherical ones. Esterification of HDL surface-bound cholesterol by the action of the enzyme lecithin cholesterol acyl transferase (LCAT) drives formed cholesteryl esters to the HDL core and maintains a gradient for free cholesterol uptake at the particle surface of maturing HDLs. At the cellular level, HDL particles engage receptors on the surface of macrophages to remove free cholesterol. The ATP-binding cassette transporters A1 and G1 (ABCA1 and ABCG1) mediate cholesterol efflux from cells to nascent and spherical HDLs. ABCA1 mainly effluxes cholesterol to nascent HDLs, and the more mature spherical HDLs target ABCG1 for cholesterol efflux. SR-B1 is a high affinity HDL receptor that mediates cellular cholesterol transport by mature spherical HDLs. SR-B1 is unique in that it can engage mature HDL species at the cell membrane and mediate cholesterol efflux, but is also responsible for taking up HDL particles and mediating the process of cellular cholesterol influx. Cellular cholesterol influx is most well understood in the context of hepatocytes as they uptake mature, spherical HDLs via SR-B1 as the last step in RCT.

Recently, nanostructures including a biomimetic nanoparticle construct with the size, shape, surface chemical composition, and cholesterol binding properties of natural, mature spherical HDL was developed, as described in International Patent Publication No. WO/2009/131704, filed April 24, 2009 and entitled, "Nanostructures Suitable for Sequestering Cholesterol and Other Molecules" and WO2011/091065 A2. Biomimetic HDLs may be synthesized using, for example, a 5 nm diameter gold nanoparticle (AuNP) as a size- and shape-restrictive template for assembling the surface chemical components of natural HDLs, including phospholipids and the HDL-defining apolipoprotein A-I (APOA-I). While International Patent Publication No. WO/2009/131704 describes the use of nanostructures for treating cancers generally, it was thought that the nanostructures could be used to treat cancer by including cancer drugs or other components that are known to be effective in killing cancer cells. In other words, it was thought that the nanostructures would be used as carriers of drugs to treat cancer. It was unexpected that the nanostructures, such as those described herein, could be used alone without the incorporation of any drugs, to treat cancer and kill cancer cells.

It was also unexpected that the nanostructures described herein could be used to control cholesterol metabolism in cells by limiting cholesterol efflux and/or influx. As described herein, in some embodiments, the nanostructures can be used to increase cholesterol efflux out of cancer cells. For example, the nanostructures, which may include a lipophilic shell and optionally Apo-A1, may be used to sequester cholesterol from the cell such that there is an efflux of cholesterol out of the cell. In some cases, the efflux of cholesterol out of the cell as a result of the nanostructures is greater than (or at least equal to) that possible with natural HDL. Furthermore, in some embodiments, the nanostructures may be used to decrease cholesterol influx into the cancer cells. Without wishing to be bound by theory, it is believed that the nanostructures described herein can be used to prevent natural HDL from binding to the cancer cells. For example, the nanostructures described herein may compete directly with natural HDL for binding with similar cell surface ligands/receptors. As noted herein, however, the nanostructures described herein may reduce proliferation and/or kill cancer cells, while natural HDL may have no such effect.

Natural HDL is used to deliver cholesterol to cancer cells, e.g., through SR-B1. This is possible in natural HDL because of the presence of esterified cholesterol in the core of the particles. Since, in some embodiments, the nanostructures described herein may include a core that does not include esterified cholesterol (e.g., a solid core that be formed of, for example, a synthetic material), the source of cholesterol to be delivered to the cancer cells can be removed. Many types of cancers depend upon HDL uptake for the delivery of cholesterol to, presumably, maintain cell membrane integrity and for cellular proliferation. Since the cells may require sufficient amounts of cholesterol to survive, treatment of cells with the nanostructures described herein may kill the cells, at least in part by decreasing the amount of cholesterol influx into the cells. Accordingly, the nanostructures described herein may be used to disrupt cholesterol metabolism in cancer cells, e.g., such that cell membrane integrity can no longer be maintained for cellular proliferation.

Little is known regarding the molecular pathways of cholesterol metabolism in cancer cells such as lymphoma cells, including the presence of molecular receptors for HDL. One aspect of the invention involves the discovery that ABCA1 and ABCG1 are expressed at relatively constant and low levels, while SR-B1 is highly expressed in multiple B-cell lymphoma cell lines, and the use of the synthetic nanostructures described herein to bind to one or more of these receptors.

In one aspect, methods for treating lymphoma cells using synthetic nanostructures are provided. The lymphoma cells may be, for example, non-Hodgkin lymphoma cells and/or B-cell lymphoma cells. In some embodiments, the lymphoma cells may be Burkitt's lymphoma cells. For example, the nanostructures described herein may inhibit Epstein Barr viral (EBV) infection of Burkitt's lymphoma. In some embodiments, the lymphoma is characterized by cells expressing SR-B1. In certain embodiments, the lymphoma is characterized by cells expressing ABCA1 and/or ABCG1. In certain embodiments, the lymphoma is characterized by cells having increased expression of SR-B1, ABCA1 and/or ABCG1. Receptors SR-B1, ABCA1 and/or ABCG1 may be used to bind to the synthetic nanostructures described herein.

In certain embodiments, the nanostructures described herein may alter membrane fluidity (e.g., of a cell membrane lipid raft). The nanostructures may influence downstream molecular pathways anchored at lipid rafts.

In certain embodiments, the nanostructures described may reduce tumor volume, size or growth (e.g., by at least 10%, at least 20%, at least 30%, at least 40%, at least 50%, at least 60%, at least 70%, at least 80%, or at least 90%) compared to the absence of treatment using the nanostructures described herein.

In general, the nanostructures described herein may have different mechanisms for treating cancer and/or killing cancer cells, including two or more different mechanisms that may occur simultaneously, and it should be appreciated that while different theories for these mechanisms have been provided, the invention is not limited in this respect. In some embodiments, the nanostructures may control the growth of cancer cells and/or kill the cancer cells. In some cases, the cancer cells may be killed by apoptosis or by other mechanisms. In certain embodiments, the nanostructures may control cholesterol metabolism in the cells (e.g., cholesterol efflux and/or cholesterol influx). In some cases, the nanostructures may modulate SR-B1 binding in cells. For example, in some embodiments the nanostructures may substantially inhibit SR-B1 (e.g., such that it is not available for binding with natural HDL). A combination of one or more such mechanisms are possible. It should be appreciated that other mechanisms are also possible.

In certain embodiments, the nanostructures described herein may bind with and/or compete for binding with the same or similar cell surface ligands/receptors as natural lipoproteins. While natural lipoproteins such as HDL (e.g., hHDL) are described predominately herein, other natural lipoproteins that the nanostructures described herein may bind with and/or compete with, such as low density lipoprotein (LDL), acetylated low density lipoprotein (Ac-LDL), intermediate density lipoprotein (IDL) and very-low-density lipoprotein (VLDL), are also possible. In some embodiments, the nanostructures described herein mimic natural lipoproteins such as LDL or VLDL. That is, the size, surface chemistry, and other features of the nanostructures may be designed to be similar to the size, surface chemistry, and other features of the natural lipoprotein.

A method of diagnosing, preventing, treating, or managing a disease or bodily condition may involve, for example, administering to a subject a therapeutically-effective amount of a composition comprising a synthetic structure described herein comprising a nanostructure core and a shell surrounding and attached to the nanostructure core, and allowing the synthetic nanostructure to bind with a cell surface ligand/receptor that binds a natural lipoprotein (e.g., HDL, IDL, LDL, or VLDL), and blocking or reducing the amount of binding between the cell surface ligand/receptor and the natural lipoprotein (e.g., compared to when the binding step of the synthetic nanostructure is not performed). The cell surface ligand/receptor may include, for example, SR-B1, ABCA1 and/or ABCG1. In some embodiments, such a method may lead to alteration of cellular cholesterol flux (e.g., increasing or decreasing cholesterol influx or efflux in a cell comprising the cell surface receptor). In certain embodiments, the synthetic nanostructure may sequester or exchange lipids or proteins (e.g., an apolipoprotein) in a biological matrix (e.g., serum, blood, plasma) with other natural lipoproteins.

In certain embodiments, a method involves administering to a biological matrix (e.g., blood, serum, plasma) a therapeutically-effective amount of a composition comprising a synthetic structure comprising a nanostructure core and a shell surrounding and attached to the nanostructure core, and allowing the synthetic nanostructure to sequester or exchange lipids or proteins with other natural lipoproteins in the biological matrix. The synthetic nanostructure may be a mimic of the natural lipoprotein. In some embodiments, the method involves binding the synthetic nanostructure to a cell surface receptor that can bind with a natural lipoprotein. The cell surface ligand/receptor may include, for example, SR-B1, ABCA1 and/or ABCG1.

In one aspect of the Present disclosure, articles, compositions, kits, and methods relating to nanostructures, including those that can be used to treat cancers or other diseases or conditions, are provided. Certain embodiments described herein include structures having a core-shell type arrangement; for instance, a nanoparticle core may be surrounded by a shell including a material, such as a lipid bilayer, that can interact with cholesterol and/or other lipids. In some embodiments, the structures, when introduced into a subject, can be used to kill cancer cells. In some cases, the nanostructures may be used to control cholesterol metabolism in cells such as cancer cells.

In some embodiments described herein, a core (e.g., a gold nanoparticle) can be used as a scaffold to template and direct the synthesis of structures of well defined size, shape, and surface chemistry that are amenable to a wide variety of further surface chemistry and tailorability. For example, a bottom-up, size-specific, lipoprotein synthesis may be carried out by using a nanostructure core to support a shell including a lipid bilayer and/or other suitable components. In some embodiments, the nanostructure core may act to restrict and template the size of formed structures and/or may be used to control cholesterol influx into cells. The shell may be used to sequester cholesterol and/or control cholesterol efflux out of cells.

Certain articles and methods described herein involve the use of nanostructure scaffolds for controllable synthesis of structures with a high degree of reproducibility and with the potential for massive scale-up. The resulting structures may be stable in a variety of solvents, may have high in vivo circulation times, and may be relatively inexpensive to fabricate. Additionally, as lipids can be easily modified with commercially available linker chemistries, the structures described herein are amenable to further functionalization with potential pharmacological agents and/or targeting/recognition agents such as antibodies, small molecules and proteins. Further advantages are described in more detail below.

Examples of nanostructures that can be used in the methods are described herein are now described.

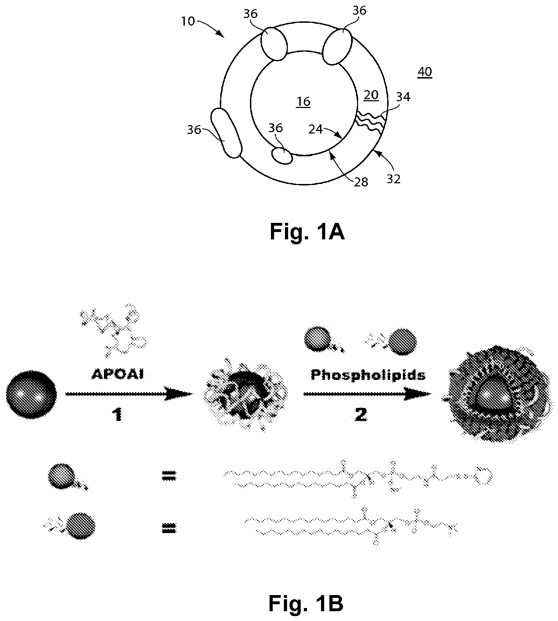

The illustrative embodiment of FIG. 1A includes a structure 10 (e.g., a synthetic structure or synthetic nanostructure) having a core 16 and a shell 20 surrounding the core. In embodiments in which the core is a nanostructure, the core includes a surface 24 to which one or more components can be optionally attached. For instance, in some cases, core 16 is a nanostructure surrounded by shell 20, which includes an inner surface 28 and an outer surface 32. The shell may be formed, at least in part, of one or more components 34, such as a plurality of lipids, which may optionally associate with one another and/or with surface 24 of the core. For example, components 34 may be associated with the core by being covalently attached to the core, physisorbed, chemisorbed, or attached to the core through ionic interactions, hydrophobic and/or hydrophilic interactions, electrostatic interactions, van der Waals interactions, or combinations thereof. In one particular embodiment, the core includes a gold nanostructure and the shell is attached to the core through a gold-thiol bond.

Optionally, components 34 can be crosslinked to one another. Crosslinking of components of a shell can, for example, allow the control of transport of species into the shell, or between an area exterior to the shell and an area interior of the shell. For example, relatively high amounts of crosslinking may allow certain small, but not large, molecules to pass into or through the shell, whereas relatively low or no crosslinking can allow larger molecules to pass into or through the shell. Additionally, the components forming the shell may be in the form of a monolayer or a multilayer, which can also facilitate or impede the transport or sequestering of molecules. In one exemplary embodiment, shell 20 includes a lipid bilayer that is arranged to sequester cholesterol and/or control cholesterol efflux out of cells, as described herein.

It should be understood that a shell which surrounds a core need not completely surround the core, although such embodiments may be possible. For example, the shell may surround at least 50%, at least 60%, at least 70%, at least 80%, at least 90%, or at least 99% of the surface area of a core. In some cases, the shell substantially surrounds a core. In other cases, the shell completely surrounds a core. The components of the shell may be distributed evenly across a surface of the core in some cases, and unevenly in other cases. For example, the shell may include portions (e.g., holes) that do not include any material in some cases. If desired, the shell may be designed to allow penetration and/or transport of certain molecules and components into or out of the shell, but may prevent penetration and/or transport of other molecules and components into or out of the shell. The ability of certain molecules to penetrate and/or be transported into and/or across a shell may depend on, for example, the packing density of the components forming the shell and the chemical and physical properties of the components forming the shell. As described herein, the shell may include one layer of material, or multilayers of materials in some embodiments.

Structure 10 (e.g., a synthetic structure or synthetic nanostructure) may also include one or more components 36 such as proteins, nucleic acids, and bioactive agents which may optionally impart specificity to the structure. One or more components 36 may be associated with the core, the shell, or both; e.g., they may be associated with surface 24 of the core, inner surface 28 of the shell, outer surface 32 of the shell, and/or embedded in the shell. For example, one or more components 36 may be associated with the core, the shell, or both through covalent bonds, physisorption, chemisorption, or attached through ionic interactions, hydrophobic and/or hydrophilic interactions, electrostatic interactions, van der Waals interactions, or combinations thereof. In one particular embodiment, shell 20 is in the form of a lipoprotein assembly or structure which includes both proteins and lipids that are covalently or non-covalently bound to one another. For example, the shell may be in the form of an apolipoprotein assembly that serves as an enzyme co-factor, receptor ligand, and/or lipid transfer carrier that regulates the uptake of lipids. As described herein, the components of structure 10 may be chosen such that the surface of the structure mimics the general surface composition of HDL, LDL, or other structures.

It should be understood that components and configurations other than those described herein may be suitable for certain structures and compositions, and that not all of the components shown in FIG. 1A are necessarily present in some embodiments.

Structure 10 may be fabricated by any suitable method. For example, in some embodiments and as shown illustratively in FIG. 1B, synthesis of a structure (e.g., a HDL-NP) may be initiated by adding apolipoprotein A-I to a solution of colloidal AuNPs (5 nm diameter). Next, one, two or more types of phospholipids may be added to the mixture. One of the phospholipids may include, for example, a di-sulfide head group which binds with high affinity to the surface of the core of the nanoparticle (e.g., gold). Other surface chemistries may also be used. A second phospholipid may be one that naturally associated with HDLs and may form the outer phospholipid leaflet layer. Fabrication methods and other details are described in U.S. Pat. No. 8,323,686, filed on April 24, 2009 and issued on December 4, 2012.

In some cases, core 16 is hollow and therefore does not include a nanostructure core. Thus, in some such and other embodiments, structure 10 includes a shell that can optionally allow components (e.g., bioactive agents, cholesterol) to pass to and from core 16 and an environment 40 outside of the shell. In contrast to certain existing hollow structures (e.g., liposomes) which typically have a largest cross-sectional dimension of greater than about 100 nm due to the steric hindrance of the components forming the shell, structures 10 having a hollow core (e.g., a partially or wholly hollow core) may be very small, e.g., having a largest cross-sectional dimension of less than about 100 nm, or even less than about 50 nm. For example, liposomes that include a lipid bilayer comprising phospholipids are difficult to fabricate having a size of less than 100 nm since the phospholipids become limited sterically, thus making it difficult or impossible to form bilayered hollow structures with small radii of curvature. Using the methods described herein, however, such and other structures having small radii of curvature can be formed, as provided herein.

In one set of embodiments, structure 10, whether including a nanostructure core or a hollow core, is constructed and arranged to sequester, transport, or exchange certain molecules to and/or from a subject or a biological sample. For instance, structure 10, when introduced into a subject, may interact with one or more components in the subject such as cells, tissues, organs, particles, fluids (e.g., blood), and portions thereof. The interaction may take place, at least in part, through the shell of structure 10, and may involve, for example, the exchange of materials (e.g., proteins, peptides, polypeptides, nucleic acids, nutrients) from the one or more components of the subject to structure 10, and/or from structure 10 to the one or more components of the subject. In some such embodiments, the shell of structure 10 can be designed to include components with properties that allow favorable interaction (e.g., binding, adsorption, transport) with the one or more materials from the subject. For example, the shell may include components having a certain hydrophobicity, hydrophilicity, surface charge, functional group, specificity for binding, and/or density to facilitate particular interactions, as described in more detail below. In certain embodiments, one or more materials from a subject are sequestered by structure 10, and structure 10 facilitates excretion, breakdown, and/or transport of the material. The excretion, breakdown, and/or transport of the material can lead to certain beneficial and/or therapeutic effects. As such, the structures described herein can be used for the diagnosis, prevention, treatment or management of certain diseases or bodily conditions.

In certain embodiments in which core 16 is not hollow, the core may comprise or be formed of a material that is toxic to cells. In some embodiments, the toxic material may be released from the core. In other embodiments, the toxic material is not released from the core. For example, contact between a component of a cell and the toxic material may affect proliferation of the cell.

In some embodiments, structure 10, whether including a nanostructure core or a hollow core, is adapted to control cholesterol metabolism and cells. For example, the nanostructure core or hollow core may be used to limit the amount of cholesterol influx into cells. This may be done by, for example, forming nanostructures that do not include any cholesterol, or sufficient amounts of cholesterol, at the core for delivery into a cell. For cells that require cholesterol for survival (e.g., to maintain membrane integrity and/or other functions), limiting the amount of cholesterol influx into cells may kill the cells. Thus, such nanostructures may be used for treatment of diseases or conditions that involve or require reducing proliferation or killing of cells. In some embodiments, structures that may be used for treatment of diseases or conditions that involve reducing proliferation and/or inducing killing of cells may include proteins (e.g., an apolipoprotein) on the surface or embedded within the shell of the structure. In other embodiments, however, no such proteins need be present on or within the shell in order for the structure to be used for treatment of diseases or conditions that involve reducing proliferation and/or inducing killing of cells.

In one particular set of embodiments, structure 10, whether including a nanostructure core or a hollow core, is constructed and arranged to sequester cholesterol (and/or other lipids). Without wishing to be bound by theory, it is hypothesized that structure 10 sequesters cholesterol through hydrophobic interactions with a hydrophobic layer (e.g., a lipid bilayer) of the structure. For example, in some cases, cholesterol can bind to a surface of the structure (e.g., to the outer surface of the shell) through hydrophobic interactions. In other cases, the cholesterol can be transported from an outer surface of the shell to an inner surface of the shell and/or to the core of the structure. The cholesterol can also be imbedded in the shell, e.g., between two layers of the shell. Optionally, structure 10 may include one or more apolipoproteins (e.g., apoliprotein-A1), proteins, or peptides, which can facilitate the sequestering of cholesterol. Structure 10 may also sequester cholesterol by removing cholesterol and phospholipids from a cell, or from other circulating lipoprotein species. Cholesterol sequestered by structure 10 may be esterified enzymatically (e.g., by lecithin:acyl CoA transferase (LCAT)) to form a cholesteryl ester that may migrate towards the center of the structure. In the case of hollow core embodiments, the cholesteryl ester may accumulate in the hollow core. In some embodiments, such and other structures may be used to control cholesterol efflux out of cells.

As described herein, the nanostructures may be used to control cholesterol metabolism in cells. For example, in some cases, the nanostructures may be used to control one or more of cholesterol influx and cholesterol influx in cells (e.g., cancer cells such as those having SR-B1, B-cell lymphoma cells, non-Hodgkin's lymphoma cells, melanoma cells and/or others). In some embodiments, the nanostructures may be used to increase cholesterol efflux out of cells, e.g., relative to natural HDL particles of similar size, shape and/or surface chemistry. Cholesterol efflux may increase by, for example, at least 1%, at least 3%, at least 5%, at least 7%, at least 10%, at least 15%, at least 20%, at least 30%, at least 40%, at least 60%, at least 80%, or at least 100% in some embodiments. Other ranges are also possible. In other cases, the nanostructures may be used to decrease cholesterol efflux out of cells. In other embodiments, the nanostructures may be used to decrease cholesterol influx into cells, e.g., relative to natural HDL particles of similar size, shape and/or surface chemistry. Cholesterol influx may decrease by, for example, at least 1%, at least 3%, at least 5%, at least 7%, at least 10%, at least 15%, at least 20%, at least 30%, at least 40%, at least 60%, at least 80%, or at least 100% in some embodiments. Other ranges are also possible. In other cases, the nanostructures may be used to increase cholesterol influx into cells. In some embodiments, the nanostructures may be used to control efflux and/or influx of cholesterol in sufficient amounts to cause the cells to significantly stop proliferating and/or to kill the cells.

In other embodiments, however, the nanostructures described herein are not constructed and arranged to sequester cholesterol.

In certain embodiments, the structures may be endocytosed by a cell (e.g., a cancer cell), thereby becoming localized within the cell. In other embodiments, the structures remain outside of a cell. Structures being both inside and outside of the cell also possible.

In some embodiments, the nanostructures described herein can sequester cholesterol from high concentrations of cholesterol and transfer it to the liver directly or indirectly. For example, cholesterol may be sequestered from areas of high concentrations of cholesterol by direct efflux of cholesterol into or onto the structures described herein. In some such embodiments, the cholesterol that is sequestered by the structures is transported directly to the liver by the structures. In other embodiments, other circulating lipoprotein species (e.g., LDL) may participate in cholesterol exchange. For example, in some cases, free cholesterol or esterified cholesterol is transferred from other lipoproteins to the structures described herein. In other cases, once free cholesterol or esterified cholesterol is sequestered by the structures described herein, the cholesterol can be transferred from the structures to the other lipoprotein species, which may ultimately end up in the liver. Thus, in such embodiments, the structures described herein can alter or augment reverse cholesterol transport indirectly. Furthermore, in the case where free cholesterol or esterified cholesterol is sequestered from the structures described herein to other lipoprotein species, the structures may further sequester cholesterol from, for example, areas of high cholesterol content, circulating lipoproteins, or other physiologic sites of high cholesterol concentration. It should be understood, however, that the structures described herein may remove cholesterol and/or other molecules by other routes, such as through urine, and the invention is not limited in this respect.

The amount of a molecule (e.g., cholesterol or other lipids) sequestered by a structure and/or a composition described herein may depend on, for example, the size of the structure, the biology and surface chemistry of the particle, as well as the method of administration. For instance, if the structures are circulated indefinitely from the periphery to the liver and out again, relative few cholesterol molecules need to be sequestered by each structure in order for the composition to be effective, since the structures are recycled. On the other hand, if a composition is used, for example, as a cholesterol or bile-salt binding resin orally, each structure may sequester a larger number of cholesterol to increased cholesterol uptake. Also, if the structures are of a size such that they are rapidly excreted (e.g., through the liver or urine) after sequestering cholesterol, a high uptake of cholesterol per structure, and/or continuous infusion may be implemented. As such, a single structure described herein, which may be incorporated into a pharmaceutical composition or other formulation, may be able to sequester any suitable number of a particular type of molecule (e.g., lipids such as cholesterol; steroids such as estrogen, progesterone, and testosterone; bile salts, etc.) during use, e.g., at least 2, at least 5, at least 10, at least 20, at least 30, at least 50, at least 100, at least 200, at least 500, at least 1,000, at least 2,000, at least 5,000, or at least 10,000 molecules, which may depend on the size (e.g., surface area and/or volume) of the structure, the particular application, and the method of administration. In some cases, such numbers of molecules can be bound to the structure at one particular instance.

In some cases, a single structure has a binding constant for cholesterol, *K*_{d}, of, for example, less than or equal to about 100 µM, less than or equal to about 10 µM, less than or equal to about 1 µM, less than or equal to about 0.1 µM, less than or equal to about 10 nM, less than or equal to about 7 nM, less than or equal to about 5 nM, less than or equal to about 2 nM, less than or equal to about 1 nM, less than or equal to about 0.1 nM, less than or equal to about 10 pM, less than or equal to about 1 pM, less than or equal to about 0.1 pM, less than or equal to about 10 fM, or less than or equal to about 1 fM. Methods for determining the amount of cholesterol sequestered and binding constants are provided in more detail below.

In certain embodiments, the molecules that are sequestered by the structures described herein cause the structure to grow in size (e.g., cross-sectional area, surface area and/or volume), e.g., depending on the number of molecules sequestered. The molecules may associate with a surface of a structure, be imbedded in a shell of a structure, be transported to a core of the structure, or combinations thereof, as described herein. As such, the size of a structure (e.g., cross-sectional area, surface area and/or volume) can increase by at least 5%, at least 10%, at least 20%, at least 30%, at least 50%, at least 70%, or at least 100%, from a time prior to sequestration compared to a time after/during sequestration in some embodiments.

It should be understood, however, that while many of the embodiments herein are described in the context of sequestering cholesterol or other lipids, the invention is not limited as such and the structures, compositions, kits, and methods described herein may be used to sequester other molecules and/or to prevent, treat, or manage other diseases or bodily conditions.

Core 16, whether being a nanostructure core or a hollow core, may have any suitable shape and/or size. For instance, the core may be substantially spherical, non-spherical, oval, rod-shaped, pyramidal, cube-like, disk-shaped, wire-like, or irregularly shaped. The core (e.g., a nanostructure core or a hollow core) may have a largest cross-sectional dimension (or, sometimes, a smallest cross-section dimension) of, for example, less than or equal to about 500 nm, less than or equal to about 250 nm, less than or equal to about 100 nm, less than or equal to about 75 nm, less than or equal to about 50 nm, less than or equal to about 40 nm, less than or equal to about 35 nm, less than or equal to about 30 nm, less than or equal to about 25 nm, less than or equal to about 20 nm, less than or equal to about 15 nm, or less than or equal to about 5 nm. In some cases, the core has an aspect ratio of greater than about 1:1, greater than 3:1, or greater than 5:1. As used herein, "aspect ratio" refers to the ratio of a length to a width, where length and width measured perpendicular to one another, and the length refers to the longest linearly measured dimension.

In embodiments in which core 16 includes a nanostructure core, the nanostructure core may be formed from any suitable material. In some embodiments, the core is formed of a synthetic material (e.g., a material that is not naturally occurring, or naturally present in the body). In one embodiment, a nanostructure core comprises or is formed of an inorganic material. The inorganic material may include, for example, a metal (e.g., Ag, Au, Pt, Fe, Cr, Co, Ni, Cu, Zn, and other transition metals), a semiconductor (e.g., silicon, silicon compounds and alloys, cadmium selenide, cadmium sulfide, indium arsenide, and indium phosphide), or an insulator (e.g., ceramics such as silicon oxide). The inorganic material may be present in the core in any suitable amount, e.g., at least 1 wt%, 5 wt%, 10 wt%, 25 wt%, 50 wt%, 75 wt%, 90 wt%, or 99 wt%. In one embodiment, the core is formed of 100 wt% inorganic material. The nanostructure core may, in some cases, be in the form of a quantum dot, a carbon nanotube, a carbon nanowire, or a carbon nanorod. In some cases, the nanostructure core comprises, or is formed of, a material that is not of biological origin. In some embodiments, a nanostructure includes or may be formed of one or more organic materials such as a synthetic polymer and/or a natural polymer. Examples of synthetic polymers include non-degradable polymers such as polymethacrylate and degradable polymers such as polylactic acid, polyglycolic acid and copolymers thereof. Examples of natural polymers include hyaluronic acid, chitosan, and collagen.

Structure 10, which may include a shell 20 surrounding core 16, may also have any suitable shape and/or size. For instance, a structure may have a shape that is substantially spherical, oval, rod-shaped, pyramidal, cubed-like, disk-shaped, or irregularly shaped. The largest cross-sectional dimension (or, sometimes, a smallest cross-section dimension) of a structure may be, for example, less than or equal to about 500 nm, less than or equal to about 250 nm, less than or equal to about 100 nm, less than or equal to about 75 nm, less than or equal to about 50 nm, less than or equal to about 40 nm, less than or equal to about 35 nm, less than or equal to about 30 nm, less than or equal to about 25 nm, less than or equal to about 20 nm, less than or equal to about 15 nm, or less than or equal to about 5 nm. The structure may also have an aspect ratio substantially similar to the aspect ratio of the core.

Furthermore, a shell of a structure can have any suitable thickness. For example, the thickness of a shell may be at least 10 Angstroms, at least 0.1 nm, at least 1 nm, at least 2 nm, at least 5 nm, at least 7 nm, at least 10 nm, at least 15 nm, at least 20 nm, at least 30 nm, at least 50 nm, at least 100 nm, or at least 200 nm (e.g., from the inner surface to the outer surface of the shell). In some cases, the thickness of a shell is less than 200 nm, less than 100 nm, less than 50 nm, less than 30 nm, less than 20 nm, less than 15 nm, less than 10 nm, less than 7 nm, less than 5 nm, less than 3 nm, less than 2 nm, or less than 1 nm (e.g., from the inner surface to the outer surface of the shell). Such thicknesses may be determined prior to or after sequestration of molecules as described herein.

Those of ordinary skill in the art are familiar with techniques to determine sizes of structures and particles. Examples of suitable techniques include dynamic light scattering (DLS) (e.g., using a Malvern Zetasizer instrument), transmission electron microscopy, scanning electron microscopy, electroresistance counting and laser diffraction. Other suitable techniques are known to those or ordinary skill in the art. Although many methods for determining sizes of nanostructures are known, the sizes described herein (e.g., largest or smallest cross-sectional dimensions, thicknesses) refer to ones measured by dynamic light scattering.

The shell of a structure described herein may comprise any suitable material, such as a hydrophobic material, a hydrophilic material, and/or an amphiphilic material. Although the shell may include one or more inorganic materials such as those listed above for the nanostructure core, in many embodiments the shell includes an organic material such as a lipid or certain polymers. The components of the shell may be chosen, in some embodiments, to facilitate the sequestering of cholesterol or other molecules. For instance, cholesterol (or other sequestered molecules) may bind or otherwise associate with a surface of the shell, or the shell may include components that allow the cholesterol to be internalized by the structure. Cholesterol (or other sequestered molecules) may also be embedded in a shell, within a layer or between two layers forming the shell.

The components of a shell may be charged, e.g., to impart a charge on the surface of the structure, or uncharged. In some embodiments, the surface of the shell may have a zeta potential of greater than or equal to about -75 mV, greater than or equal to about -60 mV, greater than or equal to about -50 mV, greater than or equal to about -40 mV, greater than or equal to about -30 mV, greater than or equal to about -20 mV, greater than or equal to about -10 mV, greater than or equal to about 0 mV, greater than or equal to about 10 mV, greater than or equal to about 20 mV, greater than or equal to about 30 mV, greater than or equal to about 40 mV, greater than or equal to about 50 mV, greater than or equal to about 60 mV, or greater than or equal to about 75 mV. The surface of the shell may have a zeta potential of less than or equal to about 75 mV, less than or equal to about 60 mV, less than or equal to about 50 mV, less than or equal to about 40mV, less than or equal to about 30 mV, less than or equal to about 20 mV, less than or equal to about 10 mV, less than or equal to about 0 mV, less than or equal to about -10 mV, less than or equal to about -20 mV, less than or equal to about -30 mV, less than or equal to about -40 mV, less than or equal to about -50 mV, less than or equal to about - 60 mV, or less than or equal to about -75 mV. Other ranges are also possible. Combinations of the above-referenced ranges are also possible (e.g., greater than or equal to about -60 mV and less than or equal to about -20 mV). As described herein, the surface charge of the shell may be tailored by varying the surface chemistry and components of the shell.

In one set of embodiments, a structure described herein or a portion thereof, such as a shell of a structure, includes one or more natural or synthetic lipids or lipid analogs (i.e., lipophilic molecules). One or more lipids and/or lipid analogues may form a single layer or a multi-layer (e.g., a bilayer) of a structure. In some instances where multi-layers are formed, the natural or synthetic lipids or lipid analogs interdigitate (e.g., between different layers). Non-limiting examples of natural or synthetic lipids or lipid analogs include fatty acyls, glycerolipids, glycerophospholipids, sphingolipids, saccharolipids and polyketides (derived from condensation of ketoacyl subunits), and sterol lipids and prenol lipids (derived from condensation of isoprene subunits).

In one particular set of embodiments, a structure described herein includes one or more phospholipids. The one or more phospholipids may include, for example, phosphatidylcholine, phosphatidylglycerol, lecithin, β, γ-dipalmitoyl-α-lecithin, sphingomyelin, phosphatidylserine, phosphatidic acid, N-(2,3-di(9-(Z)-octadecenyloxy))-prop-1-yl-N,N,N-trimethylammonium chloride, phosphatidylethanolamine, lysolecithin, lysophosphatidylethanolamine, phosphatidylinositol, cephalin, cardiolipin, cerebrosides, dicetylphosphate, dioleoylphosphatidylcholine, dipalmitoylphosphatidylcholine, dipalmitoylphosphatidylglycerol, dioleoylphosphatidylglycerol, palmitoyl-oleoyl-phosphatidylcholine, di-stearoyl-phosphatidylcholine, stearoyl-palmitoyl-phosphatidylcholine, di-palmitoyl-phosphatidylethanolamine, di-stearoyl-phosphatidylethanolamine, di-myrstoyl-phosphatidylserine, di-oleyl-phosphatidylcholine, 1,2-dipalmitoyl-sn-glycero-3-phosphothioethanol, and combinations thereof. In some cases, a shell (e.g., a bilayer) of a structure includes 50-200 natural or synthetic lipids or lipid analogs (e.g., phospholipids). For example, the shell may include less than about 500, less than about 400, less than about 300, less than about 200, or less than about 100 natural or synthetic lipids or lipid analogs (e.g., phospholipids), e.g., depending on the size of the structure.

Non-phosphorus containing lipids may also be used such as stearylamine, docecylamine, acetyl palmitate, and fatty acid amides. In other embodiments, other lipids such as fats, oils, waxes, cholesterol, sterols, fat-soluble vitamins (e.g., vitamins A, D, E and K), glycerides (e.g., monoglycerides, diglycerides, triglycerides) can be used to form portions of a structure described herein.

A portion of a structure described herein such as a shell or a surface of a nanostructure may optionally include one or more alkyl groups, e.g., an alkane-, alkene-, or alkyne-containing species, that optionally imparts hydrophobicity to the structure. An "alkyl" group refers to a saturated aliphatic group, including a straight-chain alkyl group, branched-chain alkyl group, cycloalkyl (alicyclic) group, alkyl substituted cycloalkyl group, and cycloalkyl substituted alkyl group. The alkyl group may have various carbon numbers, e.g., between C₂ and C₄₀, and in some embodiments may be greater than C₅, C₁₀, C₁₅, C₂₀, C₂₅, C₃₀, or C₃₅. In some embodiments, a straight chain or branched chain alkyl may have 30 or fewer carbon atoms in its backbone, and, in some cases, 20 or fewer. In some embodiments, a straight chain or branched chain alkyl may have 12 or fewer carbon atoms in its backbone (e.g., C₁-C₁₂ for straight chain, C₃-C₁₂ for branched chain), 6 or fewer, or 4 or fewer. Likewise, cycloalkyls may have from 3-10 carbon atoms in their ring structure, or 5, 6 or 7 carbons in the ring structure. Examples of alkyl groups include, but are not limited to, methyl, ethyl, propyl, isopropyl, cyclopropyl, butyl, isobutyl, tert-butyl, cyclobutyl, hexyl, cyclochexyl, and the like.

The alkyl group may include any suitable end group, e.g., a thiol group, an amino group (e.g., an unsubstituted or substituted amine), an amide group, an imine group, a carboxyl group, or a sulfate group, which may, for example, allow attachment of a ligand to a nanostructure core directly or via a linker. For example, where inert metals are used to form a nanostructure core, the alkyl species may include a thiol group to form a metal-thiol bond. In some instances, the alkyl species includes at least a second end group. For example, the species may be bound to a hydrophilic moiety such as polyethylene glycol. In other embodiments, the second end group may be a reactive group that can covalently attach to another functional group. In some instances, the second end group can participate in a ligand/receptor interaction (e.g., biotin/streptavidin).

In some embodiments, the shell includes a polymer. For example, an amphiphilic polymer may be used. The polymer may be a diblock copolymer, a triblock copolymer, etc., e.g., where one block is a hydrophobic polymer and another block is a hydrophilic polymer. For example, the polymer may be a copolymer of an α-hydroxy acid (e.g., lactic acid) and polyethylene glycol. In some cases, a shell includes a hydrophobic polymer, such as polymers that may include certain acrylics, amides and imides, carbonates, dienes, esters, ethers, fluorocarbons, olefins, sytrenes, vinyl acetals, vinyl and vinylidene chlorides, vinyl esters, vinyl ethers and ketones, and vinylpyridine and vinylpyrrolidones polymers. In other cases, a shell includes a hydrophilic polymer, such as polymers including certain acrylics, amines, ethers, styrenes, vinyl acids, and vinyl alcohols. The polymer may be charged or uncharged. As noted herein, the particular components of the shell can be chosen so as to impart certain functionality to the structures.

Where a shell includes an amphiphilic material, the material can be arranged in any suitable manner with respect to the nanostructure core and/or with each other. For instance, the amphiphilic material may include a hydrophilic group that points towards the core and a hydrophobic group that extends away from the core, or, the amphiphilic material may include a hydrophobic group that points towards the core and a hydrophilic group that extends away from the core. Bilayers of each configuration can also be formed.

The structures described herein may also include one or more proteins, polypeptides and/or peptides (e.g., synthetic peptides, amphiphilic peptides). In one set of embodiments, the structures include proteins, polypeptides and/or peptides that can increase the rate of cholesterol transfer or the cholesterol-carrying capacity of the structures. The one or more proteins or peptides may be associated with the core (e.g., a surface of the core or embedded in the core), the shell (e.g., an inner and/or outer surface of the shell, and/or embedded in the shell), or both. Associations may include covalent or non-covalent interactions (e.g., hydrophobic and/or hydrophilic interactions, electrostatic interactions, van der Waals interactions).

An example of a suitable protein that may associate with a structure described herein is an apolipoprotein, such as apolipoprotein A (e.g., apo A-I, apo A-II, apo A-IV, and apo A-V), apolipoprotein B (e.g., apo B48 and apo B100), apolipoprotein C (e.g., apo C-I, apo C-II, apo C-III, and apo C-IV), and apolipoproteins D, E, and H. Specifically, apo A₁, apo A₂, and apo E promote transfer of cholesterol and cholesteryl esters to the liver for metabolism and may be useful to include in structures described herein. Additionally or alternatively, a structure described herein may include one or more peptide analogues of an apolipoprotein, such as one described above. A structure may include any suitable number of, e.g., at least 1, 2, 3, 4, 5, 6, or 10, apolipoproteins or analogues thereof. In certain embodiments, a structure includes 1-6 apolipoproteins, similar to a naturally occurring HDL particle. Of course, other proteins (e.g., non-apolipoproteins) can also be included in structures described herein.

Optionally, one or more enzymes may also be associated with a structure described herein. For example, lecithin-cholesterol acyltransferase is an enzyme which converts free cholesterol into cholesteryl ester (a more hydrophobic form of cholesterol). In naturally-occurring lipoproteins (e.g., HDL and LDL), cholesteryl ester is sequestered into the core of the lipoprotein, and causes the lipoprotein to change from a disk shape to a spherical shape. Thus, structures described herein may include lecithin-cholesterol acyltransferase to mimic HDL and LDL structures. Other enzymes such as cholesteryl ester transfer protein (CETP) which transfers esterified cholesterol from HDL to LDL species may also be included.

In some embodiments, the nanostructures described herein may have a therapeutic effect in the absence of any bioactive agents. For example, in some embodiments, nanostructures or compositions thereof used to treat cancer do not include any cancer drugs (e.g., chemotherapeutic agents), and may still be effective in treating cancer cells (e.g., killing cancer cells). It should be appreciated, however, that the nanostructures and compositions described herein are not limited as such, and that in other cases, one or more bioactive agents may be associated with a nanostructure or a composition described herein. If present, the one or more bioactive agents may optionally be released from the structure or composition (e.g., long-term or short-term release). Bioactive agents include molecules that affect a biological system and include, for example proteins, nucleic acids, therapeutic agents, vitamins and their derivatives, viral fractions, lipopolysaccharides, bacterial fractions and hormones. Other agents of interest may include chemotherapeutic agents, which are used in the treatment and management of cancer patients. Such molecules are generally characterized as antiproliferative agents, cytotoxic agents and immunosuppressive agents and include molecules such as taxol, doxorubicin, daunorubicin, vinca-alkaloids, actinomycin and etoposide.

Other examples of bioactive agents include cardiovascular drugs, respiratory drugs, sympathomimetic drugs, cholinomimetic drugs, adrenergic or adrenergic neuron blocking drugs, analgesics/antipyretics, anesthetics, antiasthmatics, antibiotics, antidepressants, antidiabetics, antifungals, antihypertensives, anti-inflammatories (e.g., glucocorticoids such as prednisone), nucleic acid species (e.g., anti-sense and siRNA species against inflammatory mediators), antineoplastics, antianxiety agents, immunosuppressive agents, immunomodulatory agents, antimigraine agents, sedatives/hypnotics, antianginal agents, antipsychotics, antimanic agents, antiarrhythmics, antiarthritic agents, antigout agents, anticoagulants, thrombolytic agents, antifibrinolytic agents, hemorheologic agents, antiplatelet agents, anticonvulsants, antiparkinson agents, antihistamines/antipruritics, agents useful for calcium regulation, antibacterials, antivirals, antimicrobials, anti-infectives, bronchodialators, hypoglycemic agents, hypolipidemic agents,, agents useful for erythropoiesis stimulation, antiulcer/antireflux agents, antinauseants/antiemetics and oil-soluble vitamins, cholesterol agents (e.g., statins such as Lipitor, Zocor, which may be known to lower cholesterol levels), or combinations thereof.

In some embodiments, one or more nucleic acids is associated with a structure described herein. A nucleic acids includes any double strand or single strand deoxyribonucleic acid (DNA) or ribonucleic acid (RNA) of variable length. Nucleic acids include sense and anti-sense strands. Nucleic acid analogs such as phosphorothioates, phosphoramidates, phosphonates analogs are also considered nucleic acids and may be used. Nucleic acids also include chromosomes and chromosomal fragments.

One or more sugar residues can optionally be associated with structures described herein.

In some embodiments, the nanostructures described herein may target specific sites (e.g., specific cells and/or tissues such as cancerous cells and/or tissues) in the absence of one or more ligands or receptors specific for a particular target site or sites. For example, in some embodiments, nanostructures or compositions thereof used to treat cancer do not include any specific receptors on the nanostructure itself, and may still be effective in targeting and/or treating cancer cells (e.g., reducing the proliferation of and/or killing cancer cells). The nanostructures may specifically target a particular site (e.g., cancer cells) due to the construction of the nanostructure itself, e.g., the fact that the nanostructure mimics natural HDL in size, shape, and/or surface chemistry. It should be appreciated, however, that the nanostructures and compositions described herein are not limited as such, and that in other cases, the nanostructures may include one or more ligands or receptors specific for a particular target site or sites. For instance, a structure described herein may include a ligand for a receptor (or a receptor for a ligand) that is expressed on the surface of a site to be targeted. Examples of specific surface components include antibodies (including antibody fragments and derivatives), specific cell surface markers, small molecules (e.g., folate), and aptamers, i.e., a nucleic acid able to specifically bind a specific target molecule, such as a biological moiety (e.g., RNA aptamers and DNA aptamers). Furthermore, a protein component of the structures described herein could be modified and used as the targeting molecule, e.g. Apo E, or Apo A₁. The structures may also include certain groups (e.g., asialo groups) for targeting specific small molecules.

In other embodiments, the nanostructures described herein may target specific sites (e.g., specific cells and/or tissues such as cancerous cells and/or tissues) due to the presence of one or more ligands or receptors (e.g., an apolipoprotein, such as Apo-A1) specific for a particular target site or sites and because of the construction of the nanostructure itself, e.g., the fact that the nanostructure mimics natural HDL in size, shape, and/or surface chemistry. In some such embodiments, an equivalent nanostructure that does not include one or more ligands or receptors specific for a particular target site or sites may be somewhat effective in treating cancer cells (e.g., it may reduce the proliferation of and/or kill cancer cells to a first degree). When the targeting ligand is added to the nanostructure, then the nanostructure may be more effective in treating cancer cells, e.g., it may reduce the proliferation of and/or kill cancer cells to a second degree greater than the first degree. In some embodiments, the second degree may be at least 5%, at least 10%, at least 15%, at least 20%, at least 25%, at least 30%, at least 40%, at least 60%, or at least 80% greater than the first degree.

In other embodiments, the nanostructures described herein may include only a single type of ligand or receptor (e.g., an apolipoprotein, such as Apo-A1) that is specific for a particular target site or sites and not any other type of ligand or receptor specific for a particular target site or sites to which the nanostructure is to be administered and targeted.

In some embodiments, the nanostructures described herein do not target cancer cells specifically, but may also be directed to non-cancer cells to some extent. In some embodiments, the nanostructures may be used to kill cancer cells, or otherwise decrease proliferation of cancer cells, while having no substantial detrimental effect towards non-cancer cells (e.g., normal/peripheral human lymphocytes and/or cells from the endothelium or liver that naturally target HDL and mediate cholesterol flux). In some embodiments, the nanostructures described herein have no substantial detrimental effect towards cells that do not express SR-B1. In other embodiments, the nanostructures described herein have no substantial detrimental effect towards cells that express SR-B1 but are not cancerous (e.g., cells from the endothelium or liver that naturally target HDL and mediate cholesterol flux).

In one set of embodiments, the structures, compositions and methods described herein are used to diagnose, prevent, treat or manage diseases or bodily conditions associated with abnormal lipid levels. For instance, high density lipoprotein is a dynamic serum nanostructure protective against the development of atherosclerosis and resultant illnesses such as heart disease and stroke. By administering certain compositions and methods described herein, such as those including structures that mimic naturally occurring HDL, circulating serum HDL levels (e.g., low HDL levels) may be increased. This can provide a promising therapeutic approach to, for example, preventing and potentially reversing atherosclerosis by altering or augmenting reverse cholesterol transport. In other embodiments, the structures described herein may be used to compete directly with HDL for similar cell-surface ligands as HDL.

In yet other embodiments, compositions and methods described herein may be used to decrease LDL levels (e.g., decrease high LDL levels) or temporarily increase LDL levels, e.g., by using structure that mimics naturally occurring LDL. Furthermore, in certain embodiments, diagnosis, prevention, treatment or management of diseases or bodily conditions associated with abnormal lipid levels may involve using the structures, compositions and methods described herein to alter or augment reverse cholesterol transport (e.g., directly or indirectly) by way of altering the flux of cholesterol through and out of the body.

Other diseases or bodily conditions associated with abnormal lipid levels which could benefit from the structures and/or compositions described herein include, for example, atherosclerosis, phlebosclerosis or any venous condition in which deposits of plaques containing cholesterol or other material are formed within the intima or inner media of veins, acute coronary syndromes, angina including, stable angina, unstable angina, inflammation, sepsis, vascular inflammation, dermal inflammation, congestive heart failure, coronary heart disease (CHD), ventricular arrythmias, peripheral vascular disease, myocardial infarction, onset of fatal myocardial infarction, non-fatal myocardial infarction, ischemia, cardiovascular ischemia, transient ischemic attacks, ischemia unrelated to cardiovascular disease, ischemia-reperfusion injury, decreased need for revascularization, coagulation disorders, thrombocytopenia, deep vein thrombosis, pancreatitis, non-alcoholic steatohepatitis, diabetic neuropathy, retinopathy, painful diabetic neuropathy, claudication, psoriasis, critical limb ischemia, impotence, dyslipidemia, hyperlipidemia, hyperlipoproteinemia, hypoalphalipoproteinemia, hypertriglyceridemia, any stenotic condition leading to ischemic pathology, obesity, diabetes including both Type I and Type II, ichtyosis, stroke, vulnerable plaques, lower-limb ulceration, severe coronary ischemia, lymphomas, cataracts, endothelial dysfunction, xanthomas, end organ dysfunction, vascular disease, vascular disease that results from smoking and diabetes, carotid and coronary artery disease, regress and shrink established plaques, unstable plaques, vessel intima that is weak, unstable vessel intima, endothelial injury, endothelial damage as a result of surgical procedures, morbidity associated with vascular disease, ulcerations in the arterial lumen, restenosis as a result of balloon angioplasty, protein storage diseases (e.g., Alzheimer's disease, prion disease), diseases of hemostasis (e.g., thrombosis, thrombophilia, disseminated intravascular coagulation, thrombocytopenia, heparin induced thrombocytopenia, thrombotic thrombocytopenic purpura,), rheumatic diseases (e.g., multiple sclerosis, systemic lupus erythematosis, sjogren's syndrome, polymyositis/dermatomyositis, scleroderma), neuroligical diseases (e.g., Parkinson's disease, Alzheimer's disease), and subindications thereof.

Structures, compositions, and methods described herein may diagnose, prevent, treat, or manage diseases or bodily conditions associated with abnormal lipid levels, by, for example, decreasing triglycerides levels, increasing or decreasing the level of other lipids, increasing plaque stability or decreasing the probability of plaque rupture, increasing or decreasing vasodilation, treating or preventing inflammation, treating or preventing inflammatory diseases or an inflammatory response, strengthening or stabilizing smooth muscle and vessel intima, stimulating efflux of extracellular cholesterol for transport to the liver, modulating immune responses, mobilizing cholesterol from atherosclerotic plaques, and modifying any membrane, cell, tissue, organ, and extracellular region and/or structure in which compositional and/or functional modifications would be advantageous. In certain embodiments, the structures described herein for diagnosing, preventing, treating, or managing diseases or bodily conditions associated with abnormal lipid levels may be used to compete directly with natural lipoproteins for similar cell-surface ligands (e.g., SR-B1, HBP/vigilin).

In some embodiments, the structures described herein can be used for diagnosing, preventing, treating, or managing a disease or bodily condition by altering (e.g., increasing or decreasing) cellular cholesterol flux (e.g., efflux and influx). A method for diagnosing, preventing, treating, or managing a disease or bodily condition associated with abnormal lipid levels may involve, for example, administering to a subject a therapeutically-effective amount of a composition comprising a synthetic structure described herein comprising a nanostructure core and a shell surrounding and attached to the nanostructure core, and altering cellular cholesterol flux in the subject using the synthetic structure. As described herein, altering cellular cholesterol flux may involve binding of the structure, or a component of the structure, to one or more cell surface receptors that regulate cholesterol transport (e.g., SR-B1, ABCA1 and/or ABCG1). The disease or bodily condition associated with abnormal lipid levels may include those listed above, such as inflammation, regulation of the immune system, etc.

It should be understood that the components described herein, such as the lipids, phospholipids, alkyl groups, polymers, proteins, polypeptides, peptides, enzymes, bioactive agents, nucleic acids, and species for targeting described above (which may be optional), may be associated with a structure in any suitable manner and with any suitable portion of the structure, e.g., the core, the shell, or both. For example, one or more such components may be associated with a surface of a core, an interior of a core, an inner surface of a shell, an outer surface of a shell, and/or embedded in a shell. Furthermore, such components can be used, in some embodiments, to facilitate the sequestration, exchange and/or transport of materials (e.g., proteins, peptides, polypeptides, nucleic acids, nutrients) from one or more components of a subject (e.g., cells, tissues, organs, particles, fluids (e.g., blood), and portions thereof) to a structure described herein, and/or from the structure to the one or more components of the subject. In some cases, the components have chemical and/or physical properties that allow favorable interaction (e.g., binding, adsorption, transport) with the one or more materials from the subject.

Additionally, the components described herein, such as the lipids, phospholipids, alkyl groups, polymers, proteins, polypeptides, peptides, enzymes, bioactive agents, nucleic acids, and species for targeting described above, may be associated with a structure described herein prior to administration to a subject or biological sample and/or after administration to a subject or biological sample. For example, in some cases a structure described herein includes a core and a shell which is administered *in vivo* or *in vitro*, and the structure has a greater therapeutic effect after sequestering one or more components (e.g., an apolipoprotein) from a subject or biological sample. That is, the structure may use natural components from the subject or biological sample to increase efficacy of the structure after it has been administered.

A variety of methods can be used to fabricate the nanostructures described herein. Examples of methods are provided in International Patent Publication No. WO/2009/131704, filed April 24, 2009 and entitled, "Nanostructures Suitable for Sequestering Cholesterol and Other Molecules".

In some embodiments, because the structures described herein can be formed by the use of nanostructures that serve as a template (e.g., a core), and because certain nanostructures can be provided (e.g., made or purchased) having relatively high uniformity in size, shape, and mass, the structures described herein may also have relatively high uniformity in size, shape, and mass. That is, a mixture of relatively uniform structures can be formed, where the plurality of structures have a distribution of cross-sectional dimensions such that no more than about 20%, 15%, 10%, or 5% of the structures have a cross-sectional dimension greater than about 20%, 15%, 10%, or 5% of the average cross-sectional dimension. Structures having relatively high uniformity are useful in certain compositions described herein.

Furthermore, dispersions of structures described herein are useful in certain compositions and methods described herein.

In some cases, the structures may include or be used as contrast agents. For example, the nanostructure core of the structure may comprise a material suitable for use as a contrast agent (e.g., gold, iron oxide, a quantum dot, radionuclide, etc.). In other embodiments, the shell may include a contrast agent. For instance, a nanoparticle or other suitable contrast agent may be embedded within the lipid bilayer of the shell, or associated with an inner or outer surface of the shell. The contrast agents may be used to enhance various imaging methods known to those in the art such as MRI, X-ray, PET, CT, etc.

In other embodiments, a composition is introduced to a subject or a biological sample, and the structures of the composition and/or the subject or biological sample are exposed to assay conditions that can determine a disease or condition of the subject or biological sample. At least a portion of the structures may be retrieved from the subject or biological sample and an assay may be performed with the structures retrieved. The structures may be assayed for the amount and/or type of molecules bound to or otherwise sequestered by the structures. For example, in one set of embodiments, a competition assay is performed, e.g., where labeled cholesterol is added and displacement of cholesterol is monitored. The more measured uptake of labeled cholesterol, the less bound un-labeled free cholesterol is present. This can be done, for example, after a composition comprising the structures described herein are administered to a subject or a biological sample, and the structures are subsequently retrieved from the subject or biological sample. This method can be used, for example, where the structures are to be used as a diagnostic agent to see how much cholesterol (unlabeled) it has sequestered in a subject or biological sample.

Other methods can also be used to determine the amount of cholesterol sequestered by structures described herein. In some cases, labeled cholesterol (e.g., fluorescently-labeled cholesterol such as NBD-cholesterol, or radioactive cholesterol) can be used. Labeled cholesterol can be added to the structures either *in vitro* or *in vitro.* By adding structures without labeled cholesterol and measuring the fluorescence increase upon binding, one can calculate the binding constant of labeled cholesterol to the structure. In addition, to remove the cholesterol from the structure, one can dissolve the particle (e.g., KCN) and then measure the resultant fluorescence in solution. Comparing to standard curve can allow determination of the number of cholesterol molecules per particle. Other methods such as organic extraction and quantitative mass spectrometry can also be used to calculate amount of cholesterol sequestered by one or more structures described herein.

As described herein, the inventive structures may be used in "pharmaceutical compositions" or "pharmaceutically acceptable" compositions, which comprise a therapeutically effective amount of one or more of the structures described herein, formulated together with one or more pharmaceutically acceptable carriers, additives, and/or diluents. The pharmaceutical compositions described herein may be useful for treating cancer or other conditions. In some cases, the pharmaceutical compositions described herein may be used for killing cancer cells. Examples of cancers and cancer cells include those having scavenger receptor type B-I (SR-B1), B-cell lymphoma cells, non-Hodgkin's lymphoma cells, melanoma cells and/or others. It should be understood that any suitable structures described herein can be used in such pharmaceutical compositions, including those described in connection with the figures. In some cases, the structures in a pharmaceutical composition have a nanostructure core comprising an inorganic material and a shell substantially surrounding and attached to the nanostructure core. In some embodiments, the structures may be adapted to control cholesterol metabolism in the cells of interest, such as cholesterol influx into cells and efflux out of cells.

The pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream or foam; sublingually; ocularly; transdermally; or nasally, pulmonary and to other mucosal surfaces.

The phrase "pharmaceutically acceptable" is employed herein to refer to those structures, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

The structures described herein may be orally administered, parenterally administered, subcutaneously administered, and/or intravenously administered. In certain embodiments, a structure or pharmaceutical preparation is administered orally. In other embodiments, the structure or pharmaceutical preparation is administered intravenously. Alternative routes of administration include sublingual, intramuscular, and transdermal administrations.

Pharmaceutical compositions described herein include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, and the particular mode of administration. The amount of active ingredient that can be combined with a carrier material to produce a single dosage form will generally be that amount of the compound which produces a therapeutic effect. Generally, this amount will range from about 1% to about 99% of active ingredient, from about 5% to about 70%, or from about 10% to about 30%.

The inventive compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a structure described herein as an active ingredient. An inventive structure may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol, glycerol monostearate, and non-ionic surfactants; absorbents, such as kaolin and bentonite clay; lubricants, such as talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made in a suitable machine in which a mixture of the powdered structure is moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the structures described herein include pharmaceutically acceptable emulsions, microemulsions, solutions, dispersions, suspensions, syrups and elixirs. In addition to the inventive structures, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions described herein (e.g., for rectal or vaginal administration) may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the body and release the structures.

Dosage forms for the topical or transdermal administration of a structure described herein include powders, sprays, ointments, pastes, foams, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to the inventive structures, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the structures described herein, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a structure described herein to the body. Dissolving or dispersing the structure in the proper medium can make such dosage forms. Absorption enhancers can also be used to increase the flux of the structure across the skin. Either providing a rate controlling membrane or dispersing the structure in a polymer matrix or gel can control the rate of such flux.

Ophthalmic formulations, eye ointments, powders, solutions and the like, are also contemplated as being within the scope of this invention.

Pharmaceutical compositions described herein suitable for parenteral administration comprise one or more inventive structures in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the inventive structures may be facilitated by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Delivery systems suitable for use with structures and compositions described herein include time-release, delayed release, sustained release, or controlled release delivery systems, as described herein. Such systems may avoid repeated administrations of the structures in many cases, increasing convenience to the subject and the physician. Many types of release delivery systems are available and known to those of ordinary skill in the art. They include, for example, polymer based systems such as polylactic and/or polyglycolic acid, polyanhydrides, and polycaprolactone; nonpolymer systems that are lipid-based including sterols such as cholesterol, cholesterol esters, and fatty acids or neutral fats such as mono-, di- and triglycerides; hydrogel release systems; silastic systems; peptide based systems; wax coatings; compressed tablets using conventional binders and excipients; or partially fused implants. Specific examples include, but are not limited to, erosional systems in which the composition is contained in a form within a matrix, or diffusional systems in which an active component controls the release rate. The compositions may be as, for example, microspheres, hydrogels, polymeric reservoirs, cholesterol matrices, or polymeric systems. In some embodiments, the system may allow sustained or controlled release of the active compound to occur, for example, through control of the diffusion or erosion/degradation rate of the formulation. In addition, a pump-based hardware delivery system may be used in some embodiments. The structures and compositions described herein can also be combined (e.g., contained) with delivery devices such as syringes, pads, patches, tubes, films, MEMS-based devices, and implantable devices.

Use of a long-term release implant may be particularly suitable in some cases. "Long-term release," as used herein, means that the implant is constructed and arranged to deliver therapeutic levels of the composition for at least about 30 or about 45 days, for at least about 60 or about 90 days, or even longer in some cases. Long-term release implants are well known to those of ordinary skill in the art, and include some of the release systems described above.

Injectable depot forms can be made by forming microencapsule matrices of the structures described herein in biodegradable polymers such as polylactide-polyglycolide. Depending on the ratio of structure to polymer, and the nature of the particular polymer employed, the rate of release of the structure can be controlled. Examples of other biodegradable polymers include poly(orthoesters) and poly(anhydrides).

When the structures described herein are administered as pharmaceuticals, to humans and animals, they can be given per se or as a pharmaceutical composition containing, for example, about 0.1% to about 99.5%, about 0.5% to about 90%, or the like, of structures in combination with a pharmaceutically acceptable carrier.

The administration may be localized (e.g., to a particular region, physiological system, tissue, organ, or cell type) or systemic, depending on the condition to be treated. For example, the composition may be administered through parental injection, implantation, orally, vaginally, rectally, buccally, pulmonary, topically, nasally, transdermally, surgical administration, or any other method of administration where access to the target by the composition is achieved. Examples of parental modalities that can be used with the invention include intravenous, intradermal, subcutaneous, intracavity, intramuscular, intraperitoneal, epidural, or intrathecal. Examples of implantation modalities include any implantable or injectable drug delivery system. Oral administration may be useful for some treatments because of the convenience to the patient as well as the dosing schedule.

Regardless of the route of administration selected, the structures described herein, which may be used in a suitable hydrated form, and/or the inventive pharmaceutical compositions, are formulated into pharmaceutically-acceptable dosage forms by conventional methods known to those of skill in the art.

The compositions described herein may be given in dosages, e.g., at the maximum amount while avoiding or minimizing any potentially detrimental side effects. The compositions can be administered in effective amounts, alone or in a combinations with other compounds. For example, when treating cancer, a composition may include the structures described herein and a cocktail of other compounds that can be used to treat cancer. When treating conditions associated with abnormal lipid levels, a composition may include the structures described herein and other compounds that can be used to reduce lipid levels (e.g., cholesterol lowering agents).

The phrase "therapeutically effective amount" as used herein means that amount of a material or composition comprising an inventive structure which is effective for producing some desired therapeutic effect in a subject at a reasonable benefit/risk ratio applicable to any medical treatment. Accordingly, a therapeutically effective amount may, for example, prevent, minimize, or reverse disease progression associated with a disease or bodily condition. Disease progression can be monitored by clinical observations, laboratory and imaging investigations apparent to a person skilled in the art. A therapeutically effective amount can be an amount that is effective in a single dose or an amount that is effective as part of a multi-dose therapy, for example an amount that is administered in two or more doses or an amount that is administered chronically.

The effective amount of any one or more structures described herein may be from about 10 ng/kg of body weight to about 1000 mg/kg of body weight, and the frequency of administration may range from once a day to once a month. However, other dosage amounts and frequencies also may be used as the invention is not limited in this respect. A subject may be administered one or more structure described herein in an amount effective to treat one or more diseases or bodily conditions described herein.

An effective amount may depend on the particular condition to be treated. One of ordinary skill in the art can determine what an effective amount of the composition is by, for example, methods such as assessing liver function tests (e.g. transaminases), kidney function tests (e.g. creatinine), heart function tests (e.g. troponin, CRP), immune function tests (e.g. cytokines like IL-1 and TNF-alpha), etc. The effective amounts will depend, of course, on factors such as the severity of the condition being treated; individual patient parameters including age, physical condition, size and weight; concurrent treatments; the frequency of treatment; or the mode of administration. These factors are well known to those of ordinary skill in the art and can be addressed with no more than routine experimentation. In some cases, a maximum dose be used, that is, the highest safe dose according to sound medical judgment.

Actual dosage levels of the active ingredients in the pharmaceutical compositions described herein may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular inventive structure employed, the route of administration, the time of administration, the rate of excretion or metabolism of the particular structure being employed, the duration of the treatment, other drugs, compounds and/or materials used in combination with the particular structure employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the structures described herein employed in the pharmaceutical composition at levels lower than that required to achieve the desired therapeutic effect and then gradually increasing the dosage until the desired effect is achieved.

In some embodiments, a structure or pharmaceutical composition described herein is provided to a subject chronically. Chronic treatments include any form of repeated administration for an extended period of time, such as repeated administrations for one or more months, between a month and a year, one or more years, or longer. In many embodiments, a chronic treatment involves administering a structure or pharmaceutical composition repeatedly over the life of the subject. For example, chronic treatments may involve regular administrations, for example one or more times a day, one or more times a week, or one or more times a month. In general, a suitable dose such as a daily dose of a structure described herein will be that amount of the structure that is the lowest dose effective to produce a therapeutic effect. Such an effective dose will generally depend upon the factors described above. Generally doses of the structures described herein for a patient, when used for the indicated effects, will range from about 0.0001 to about 100 mg per kg of body weight per day. The daily dosage may range from 0.001 to 50 mg of compound per kg of body weight, or from 0.01 to about 10 mg of compound per kg of body weight. However, lower or higher doses can be used. In some embodiments, the dose administered to a subject may be modified as the physiology of the subject changes due to age, disease progression, weight, or other factors.

If desired, the effective daily dose of the active compound may be administered as two, three, four, five, six or more sub-doses administered separately at appropriate intervals throughout the day, optionally, in unit dosage forms. For example, instructions and methods may include dosing regimens wherein specific doses of compositions, especially those including structures described herein having a particular size range, are administered at specific time intervals and specific doses to achieve reduction of cholesterol (or other lipids) and/or treatment of disease while reducing or avoiding adverse effects or unwanted effects. Thus, methods of administering structures described herein, methods of reducing total and LDL cholesterol by the administration of the structures, methods of raising the level or increasing the efficacy of HDL cholesterol by the administration of structures described herein, and methods of dosing structures in patients in need thereof are described.

While it is possible for a structure described herein to be administered alone, it may be administered as a pharmaceutical composition as described above. The present invention also provides any of the above-mentioned compositions useful for diagnosing, preventing, treating, or managing a disease or bodily condition packaged in kits, optionally including instructions for use of the composition. That is, the kit can include a description of use of the composition for participation in any disease or bodily condition, including those associated with abnormal lipid levels. The kits can further include a description of use of the compositions as discussed herein. The kit also can include instructions for use of a combination of two or more compositions described herein. Instructions also may be provided for administering the composition by any suitable technique, such as orally, intravenously, or via another known route of drug delivery.

The kits described herein may also contain one or more containers, which can contain components such as the structures, signaling entities, and/or biomolecules as described. The kits also may contain instructions for mixing, diluting, and/or administrating the compounds. The kits also can include other containers with one or more solvents, surfactants, preservatives, and/or diluents (e.g., normal saline (0.9% NaCl), or 5% dextrose) as well as containers for mixing, diluting or administering the components to the sample or to the patient in need of such treatment.

The compositions of the kit may be provided as any suitable form, for example, as liquid solutions or as dried powders. When the composition provided is a dry powder, the powder may be reconstituted by the addition of a suitable solvent, which may also be provided. In embodiments where liquid forms of the composition are used, the liquid form may be concentrated or ready to use. The solvent will depend on the particular inventive structure and the mode of use or administration. Suitable solvents for compositions are well known and are available in the literature.

The kit, in one set of embodiments, may comprise one or more containers such as vials, tubes, and the like, each of the containers comprising one of the separate elements to be used in the method. For example, one of the containers may comprise a positive control in the assay. Additionally, the kit may include containers for other components, for example, buffers useful in the assay.

As used herein, a "subject" or a "patient" refers to any mammal (e.g., a human), for example, a mammal that may be susceptible to a disease or bodily condition such as a disease or bodily condition associated with abnormal lipid levels. Examples of subjects or patients include a human, a non-human primate, a cow, a horse, a pig, a sheep, a goat, a dog, a cat or a rodent such as a mouse, a rat, a hamster, or a guinea pig. Generally, the invention is directed toward use with humans. A subject may be a subject diagnosed with a certain disease or bodily condition or otherwise known to have a disease or bodily condition. In some embodiments, a subject may be diagnosed as, or known to be, at risk of developing a disease or bodily condition. In some embodiments, a subject may be diagnosed with, or otherwise known to have, a disease or bodily condition associated with abnormal lipid levels, as described herein. In certain embodiments, a subject may be selected for treatment on the basis of a known disease or bodily condition in the subject. In some embodiments, a subject may be selected for treatment on the basis of a suspected disease or bodily condition in the subject. In some embodiments, the composition may be administered to prevent the development of a disease or bodily condition. However, in some embodiments, the presence of an existing disease or bodily condition may be suspected, but not yet identified, and a composition of the invention may be administered to diagnose or prevent further development of the disease or bodily condition.

A "biological sample," as used herein, is any cell, body tissue, or body fluid sample obtained from a subject. Non-limiting examples of body fluids include, for example, lymph, saliva, blood, urine, and the like. Samples of tissue and/or cells for use in the various methods described herein can be obtained through standard methods including, but not limited to, tissue biopsy, including punch biopsy and cell scraping, needle biopsy; or collection of blood or other bodily fluids by aspiration or other suitable methods.

The following examples are intended to illustrate certain embodiments of the present invention, but are not to be construed as limiting and do not exemplify the full scope of the invention.

### EXAMPLES

This example demonstrates the use of synthetic nanostructures (e.g., HDL-NPs) to treat cancers, such as cancers expressing SR-B1.

Functional, biomimetic high-density lipoprotein nanoparticle (HDL-NP) as a potential therapy for B-cell lymphoma are described. The HDL-NPs appear to function through their ability to target scavenger receptor type B-1 (SR-B1) and their functional capacity for cholesterol sequestration. As such, the HDL-NP is a surface chemical mimic of spherical HDLs; however, a gold nanoparticle template used to control the size and shape of the HDL-NP occupies some of the particle real estate typically reserved for esterified cholesterol. Thus, like natural HDL, the HDL-NP engages SR-B1, but provides minimal cholesterol to cells. At the same time, the HDL-NP sequesters free cellular cholesterol on its surface. The data herein demonstrate that HDL-NPs target SR-B1 expressed by B-cell lymphoma cell lines, maximally efflux cholesterol when compared to natural HDLs, and induce apoptosis. Serum-derived HDLs and Ac-LDL do not induce apoptosis. Accordingly, a functional, synthetic single-entity therapeutic nanostructure that derives therapeutic benefit through biomimicry and control over the inherent biological function of the nanoparticle has been demonstrated.

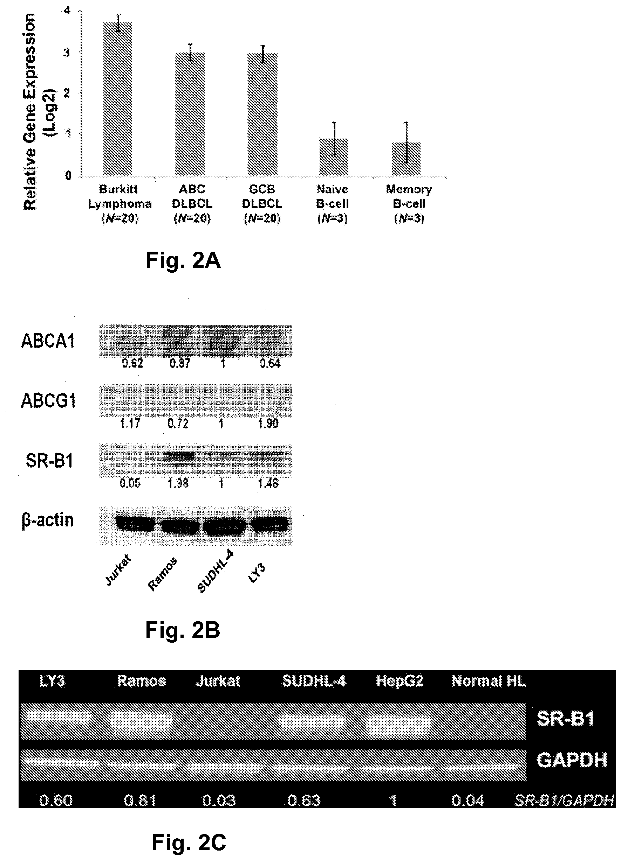

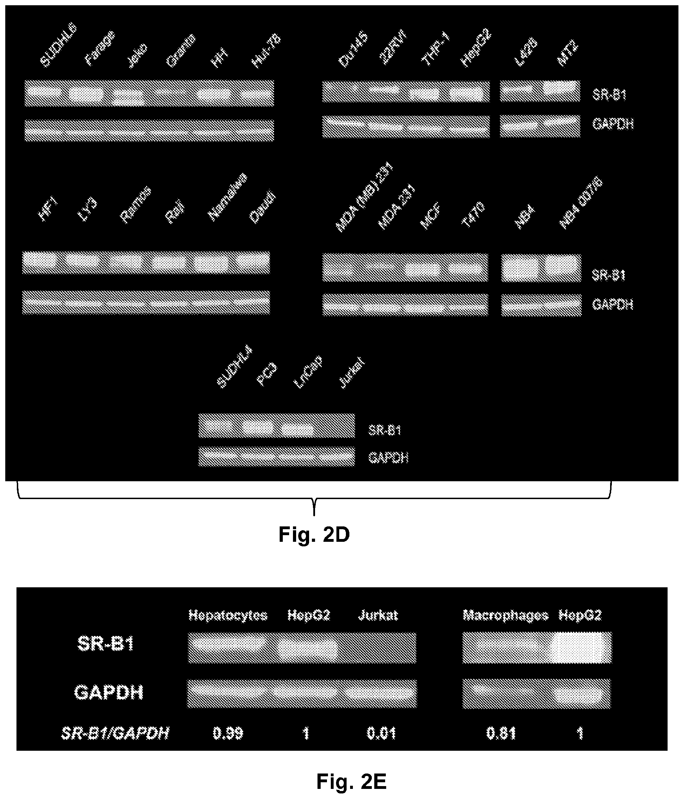

**Expression of SR-B1 in lymphoma and in cell lines.** Little is known about the molecular pathways of cholesterol metabolism in lymphoma, including the prevalence of receptors for the uptake of cholesterol-rich HDLs. Consequently, we examined gene expression profiles of DLBCL (ABC-like and GC-like), Burkitt's lymphoma (BL) and normal B cells from human samples in a database generated using Affymetrix U133plus 2.0 arrays (FIG. 2A). We found that SR-B1 was expressed at -9-16 times the level in the lymphomas as compared to normal B cells. Next, we determined the expression of the SR-B1 protein in lymphoma cell lines and normal human peripheral lymphocytes by immunoblotting (FIG. 2E and 2C). We found that ABCA1 and ABCG1 are expressed at relatively constant and low levels, while SR-B1 is highly expressed in multiple B-cell lymphoma cell lines (FIG. 2B). Interestingly, SR-B1 is not expressed in Jurkat - a lymphoma cell line of T-cell lineage (FIG. 2B). SR-B1 is expressed in multiple B cell lymphoma cell lines, but not in normal human lymphocytes or Jurkat, a lymphoma cell line of T-cell lineage (FIG. 2C). HepG2 liver hepatoma cells, known to express SR-B, were included for comparison. Finally, Western blot profiling revealed that SR-B1 is expressed in multiple cancer cell lines (FIG. 2E) as well as well as hepatocytes and macrophages (FIG. 2F).

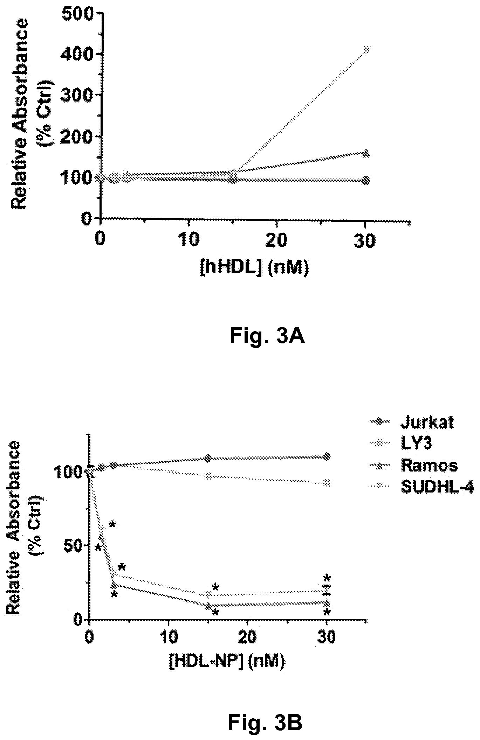

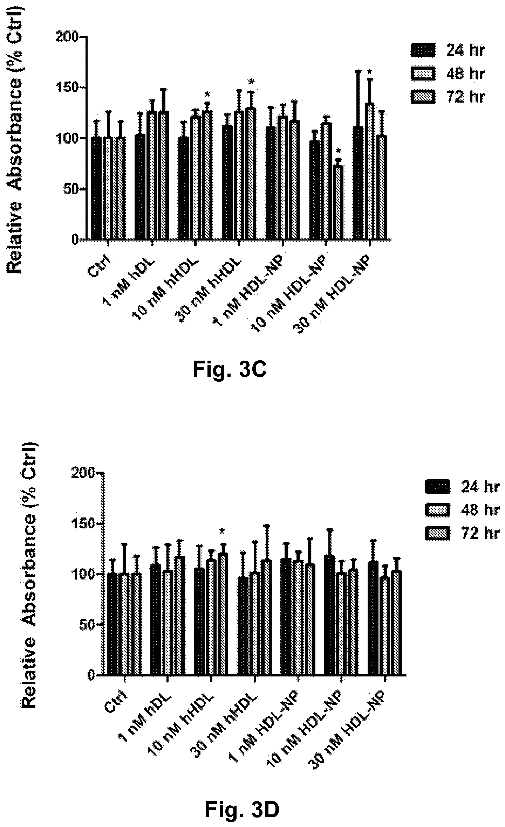

**Cell viability in lymphoma cell lines after exposure to HDL-NPs.** Ramos and SUDHL-4 cell lines are GC-derived B cell lines from BL and DLBCL, respectively. In addition, we chose to study the ABC-like DLBCL line, LY3. Jurkat cells and normal human lymphocytes provided SR-B1 receptor negative controls. In addition, we also chose two primary cells known to express SR-B1 that are critical cell types naturally engaged by HDLs, hepatocytes and macrophages. For each of the cell types, we measured cell viability by an MTS assay following treatment with human serum derived HDL (hHDL) or HDL-NPs. MTS is a colorimetric assay where the magnitude of absorbance is proportional to cell viability. For each treatment, and for each comparison made throughout our studies, we added equivalent doses of hHDL and HDL-NPs based upon the amount of apolipoprotein A-1 (Apo A1). Addition of hHDL did not change the relative absorbance values measured using the MTS assay for LY3 or Jurkat cells, but increased for Ramos and SUDHL-4 cells (FIG. 3A). Conversely, treatment with HDL-NPs resulted in a dose-dependent decrease in absorbance obtained in the Ramos and SUDHL-4 cells, less so in LY3 cells, and not in the Jurkat line (FIG. 3B). Treatment with hHDL or HDL-NPs had no effect in primary hepatocytes or macrophage cells (FIGs. 3C and 3D). Thus, in direct contrast to their natural hHDL counterparts, HDL-NPs selectively reduce the viability of GC- and ABC-derived lymphoma cells and spare Jurkat, primary human hepatocytes, and primary human macrophages.

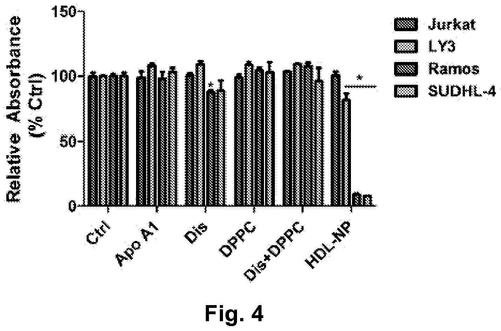

**Biomimicry of HDL-NP.** In order to determine the influence of the free chemical components of HDL-NPs from the synthetic HDL-NP constructs, each (i.e. Apo A1 and phospholipids) was added to Ramos, SUDHL-4, LY3, and Jurkat cells and MTS assays were performed. The free components had no significant effect, except for a relatively small but statistically significant reduction in the absorbance value measured after adding the disulfide-containing lipid (PDP PE) to Ramos cells (FIG. 4). These data demonstrate that the toxicity of the HDL-NP toward sensitive B cell lines is derived through biomimicry, rather than any toxic effects of the individual components of the HDL-NP.

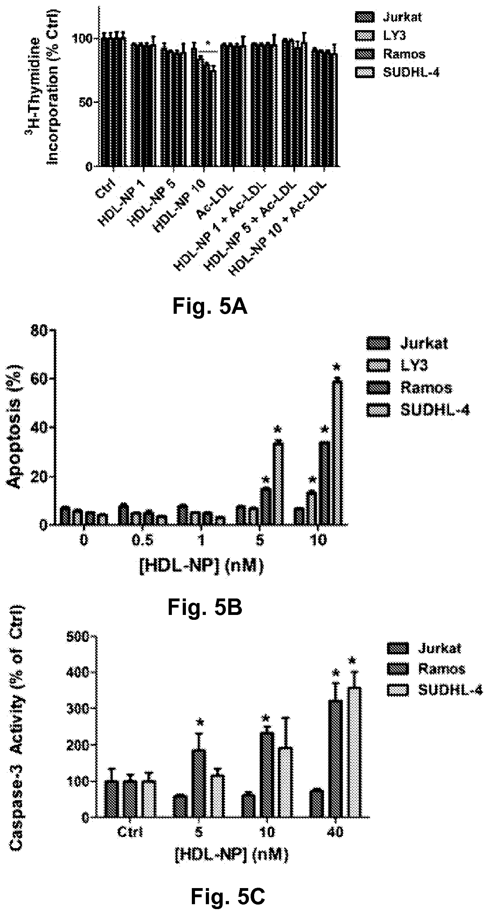

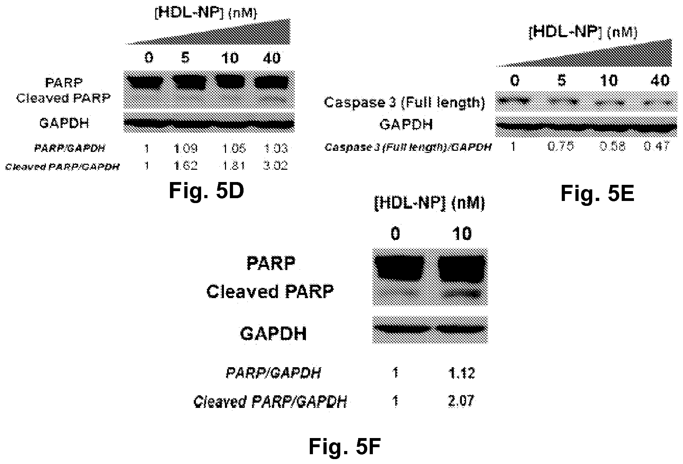

**Apoptosis in lymphoma cell lines.** Because changes in absorbance measured with an MTS assay can be multi-factorial, we also measured cellular apoptosis and proliferation (FIG. 5A) after treatment with hHDL and HDL-NPs. Using Annexin V and propidium iodide cell labeling and flow cytometry (see Materials and Methods) we found that HDL-NPs induced dose- and time-dependent apoptosis in B cell lymphoma cell lines (FIG. 5B) while sparing Jurkat (FIG. 5B). At the molecular level, our data demonstrate that HDL-NPs cause a dose-dependent increase in cleaved poly-ADP ribose polymerase (PARP) and a reduction in full-length caspase 3 levels in Ramos cells (FIGS. 5D and 5E). In SUDHL-4 cells, cleaved PARP levels begin to increase 24-hr after treatment with HDL-NPs (FIG. 5F). In addition, using a colorimetric assay of activated caspase 3 activity (see Materials and Methods), we found that HDL-NP treatment induces a time- and dose-dependent increase in activated caspase 3 activity in Ramos and SUDHL-4 cells, but not in Jurkat cells (FIG. 5C).

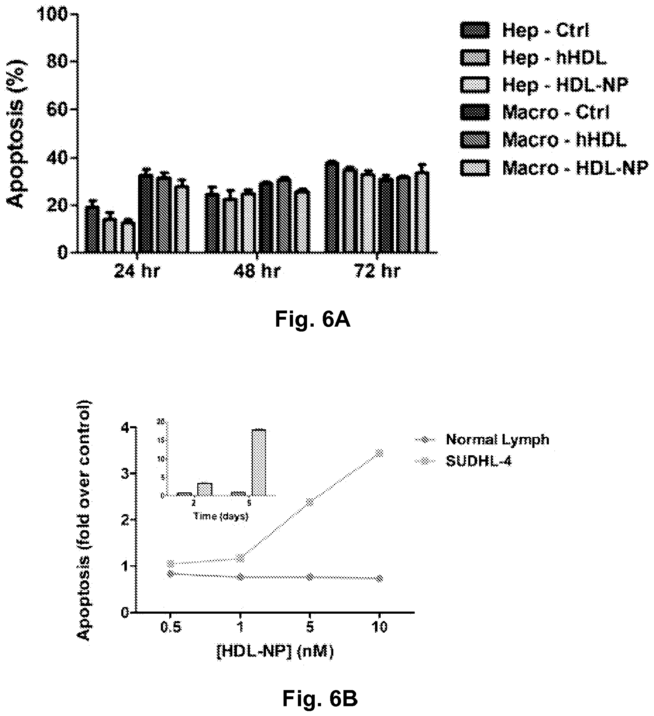

**Investigation of normal hepatocytes, macrophages and lymphocytes.** Next, we measured the toxicity of HDL-NPs to normal hepatocytes and macrophages (FIG. 6A), as well as to naive human lymphocytes (FIG. 6B). First, apoptosis was measured after exposing normal human hepatocytes and macrophages to hHDL and HDL-NPs for 24, 48, and 72 hrs. No increase in apoptosis was observed for treated versus control cells (FIG. 6A). Next, blood from a human volunteer was collected and lymphocytes were isolated using a Ficoll gradient. Normal human lymphocytes did not undergo apoptosis when treated for 72 hours with increasing doses of HDL-NP (FIG. 6B) or after exposure to 10 nM HDL-NPs, a dose toxic to SUDHL-4 and Ramos cells, at 48 hours and 5 days (FIG. 6B, inset). Collectively, these data demonstrate that the HDL-NPs are not toxic to cells normally targeted by HDL *in vivo* or nucleated cells normally found in blood.

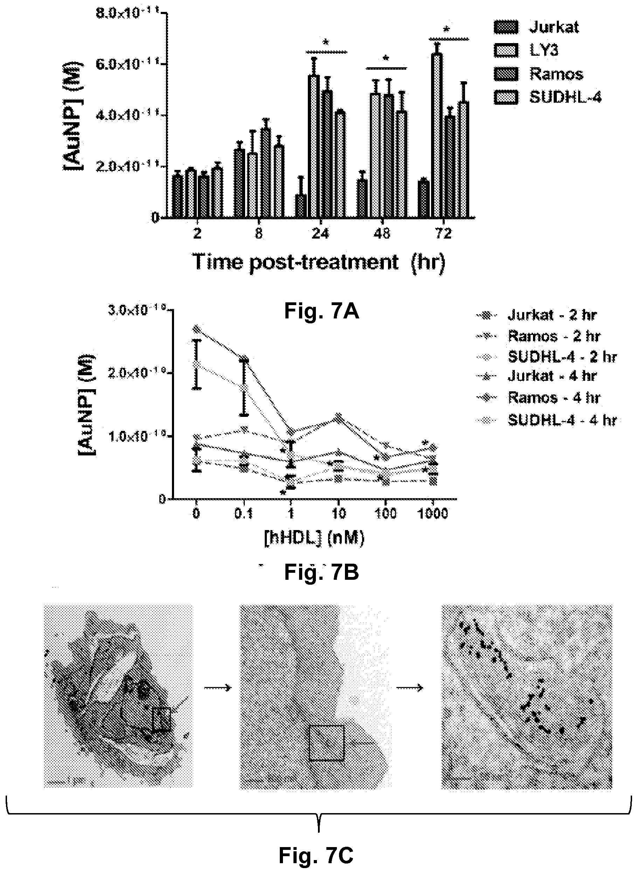

**Engagement of SR-B1 by HDL-NP and rescue by native HDL and Ac-LDL.** We reasoned that apoptosis induction was related to SR-B1 engagement by HDL-NPs mimicking uptake of mature, cholesterol-rich HDLs. We measured gold content by inductively coupled plasma mass spectrometry (ICP-MS) and correlated cellular gold content with cellular SR-B1 expression (FIG. 7A). Measurements of cellular gold content are normalized to cellular protein and at later time points are a combination of live and apoptotic cells (72 hrs). Mass spectrometry data indicate that HDL-NPs were initially engaged with cells at two hours, followed by an increase in cellular gold content in Ramos, SUDHL-4, and LY3 cells (but not in Jurkat) until a saturation plateau was reached at 24 hours. Collectively, these data are consistent with measured SR-B 1 expression by these cell types. Further, in order to understand if natural hHDLs compete with HDL-NPs for the same engagement and uptake mechanisms in each of the cell types, we performed a competition experiment with increasing concentrations of hHDL. Data were collected at early time points (*t* = 2 and 4 hrs) in order to isolate, and potentially inhibit, early cell binding. Data demonstrate that as hHDL concentrations increase, cellular gold content steadily decreases in Ramos and SUDHL-4 cells (FIG. 7B). There is relatively scant uptake by SR-B1 negative Jurkat cells at both time points (FIG. 7B). Next, we used transmission electron microscopy (TEM) to visualize HDL-NP engagement and uptake in SUDHL-4 cells (FIG. 7C). Micrographs demonstrate AuNP uptake by SUDHL-4 cells after HDL-NP treatment. At the sub-cellular level, AuNPs were restricted to the cell membrane, cytoplasm, and vesicular structures as shown in FIG. 7C. No AuNPs were observed in cell nuclei. Taken together, these data suggest that HDL-NPs compete with hHDLs for SR-B1 and can be internalized by target cells.

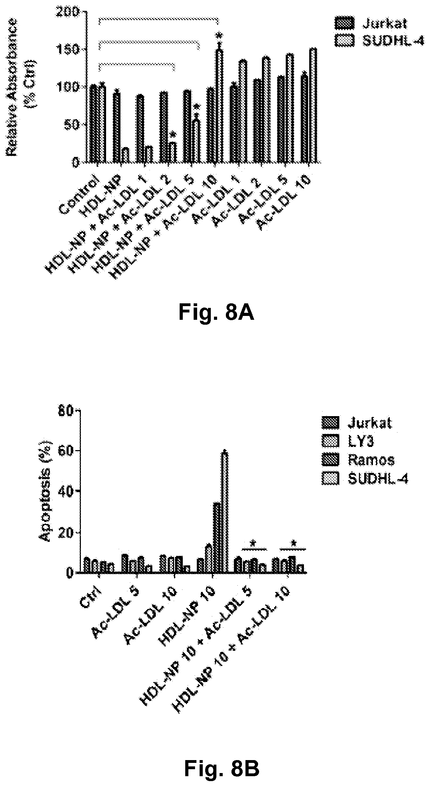

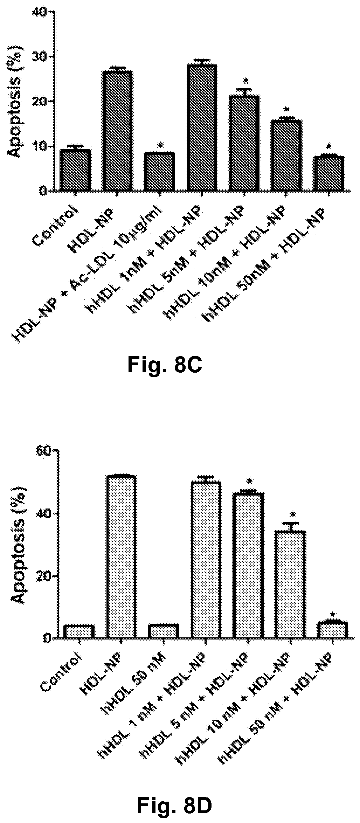

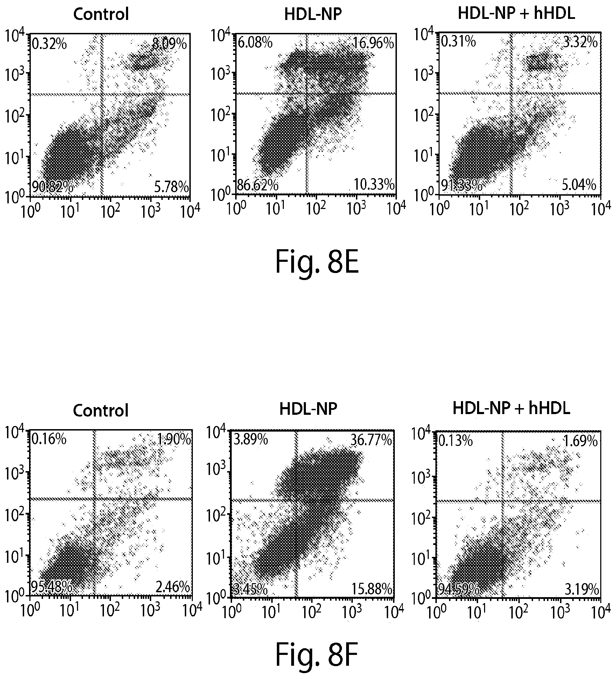

To explore the role of SR-B1 engagement and to better understand if cholesterol flux contributes to apoptosis induction following HDL-NP cell treatment, we performed a rescue experiment by adding known SR-B 1 particulate agonists that are also a source of cholesterol. Acetylated low-density lipoprotein (Ac-LDL) and hHDL both utilize SR-B1 to deliver cholesterol to cells. We measured viability and apoptosis in the presence of increasing concentrations of Ac-LDL while keeping the HDL-NP concentration constant and at a dose toxic to Ramos and SUDHL-4 cells (10 nM). Absorbance data obtained using the MTS assay demonstrate that SUDHL-4 cells were rescued by adding an increasing concentration of Ac-LDL (FIG. 8A). No change was observed for Jurkat cells (FIG.8A). Further, both Ac-LDL (FIG. 8B) and hHDL (FIGS. 8C-8F) rescued Ramos and SUDHL-4 cells, but not Jurkat (FIG. 8B), from HDL-NP-mediated apoptosis in a dose-dependent manner. Because changes in cell proliferation can confound data provided by MTS cell viability assays, we evaluated ³H-thymidine incorporation as a measure of cell proliferation in all four cell lines (FIG. 5A). Our data demonstrate that HDL-NPs mildly reduced cellular proliferation in LY3, Ramos, and SUDHL-4 cell lines, but not in SR-B1 negative Jurkat cells. The addition of Ac-LDL rescued cellular proliferation to baseline levels but did not induce significant cell proliferation in any of the tested cell lines when added alone (FIG. 5A). Therefore, HDL-NPs target SR-B1, induce apoptosis, and mildly reduce cell proliferation by altering cholesterol flux through this receptor.

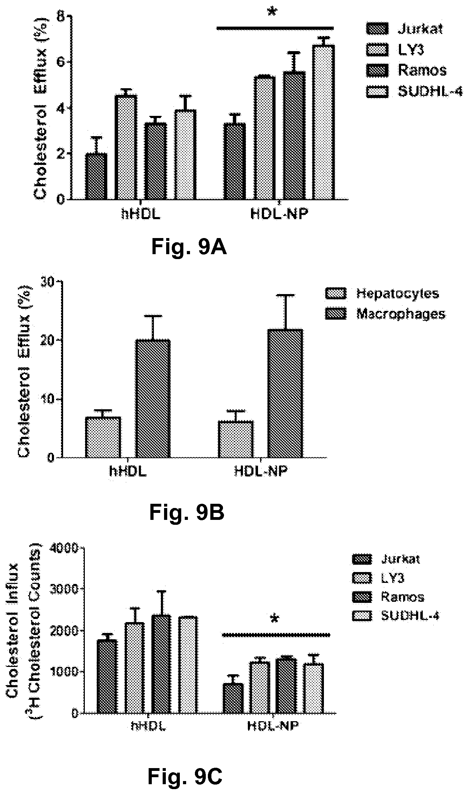

**Measurements of cholesterol flux.** Owing to the potential for SR-B1 to mediate both cholesterol influx and efflux, we measured cholesterol flux in cell lines and primary cells in the presence of hHDL and HDL-NPs (FIGS. 9A-9F). In the lymphoma cell lines, cholesterol efflux was greatest after exposure to the HDL-NPs (FIG. 9A). Jurkat cells demonstrated the least amount of cholesterol efflux. In normal cells, measured cholesterol efflux was higher in macrophages than in hepatocytes and the magnitude of efflux was similar for hHDL and HDL-NPs (FIG. 9B).

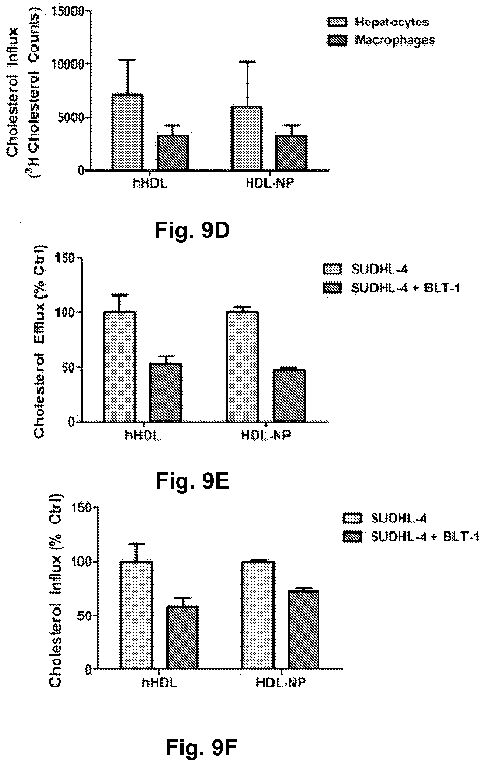

Next, we determined the capacity of hHDL and HDL-NPs to influx cholesterol to cultured lymphoma cells (FIG. 9C) and normal human hepatocytes and macrophages (FIG. 9D). Compared with hHDL, HDL-NPs delivered the least amount of cholesterol to each of the tested lymphoma cell lines (FIG. 9C). In normal cells, cholesterol influx was greatest in hepatocytes versus macrophages and the magnitude was relatively equal to hHDL and HDL-NPs (FIG. 9D). Taken together, HDL-NPs appear to differentially modulate cholesterol flux in the lymphoma cell lines as opposed to the normal cells where flux appears more evenly controlled. Combining the cell death and cholesterol flux data provides evidence that the mechanism-of-action of the HDL-NPs is derived from differential manipulation of cellular cholesterol metabolism and molecular pathways downstream of SR-B1.

**Inhibition of SR-B1 by BLT-1 blocks cholesterol flux to HDL-NPs.** Blocker of lipid transport-1 (BLT-1) is a small molecule that binds cysteine-384 in the extracellular loop domain of SR-B1 and inhibits cholesterol flux through SR-B1 without altering the binding of HDL particles to the receptor. Thus, treatment of SUDHL-4 cells with BLT-1 allowed a measurement of engagement and cholesterol flux through SR-B1. Our data demonstrate that BLT-1 inhibited cholesterol flux to hHDL and HDL-NPs providing evidence that engagement of SR-B1 by HDL-NPs is responsible for altering cholesterol flux and consistent with previous reports (FIGs. 9E and 9F).

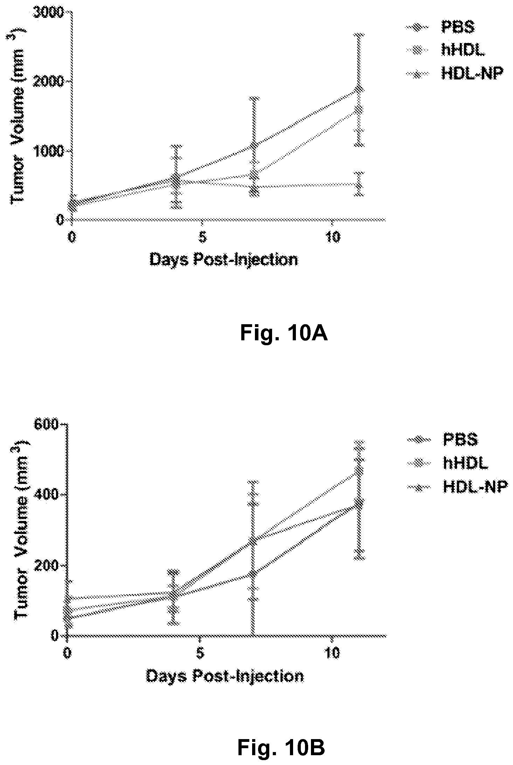

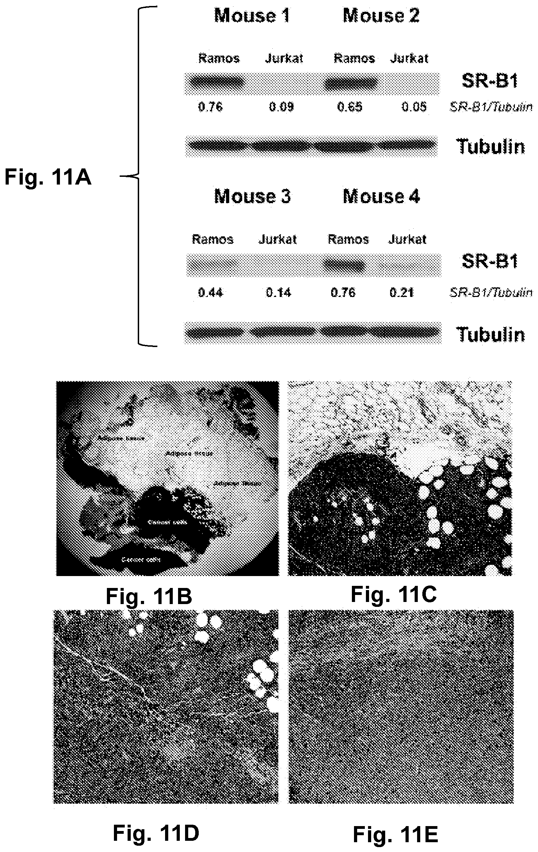

**Lymphoma xenograft experiments.** To recapitulate our *in vitro* data in an *in vivo* model, we administered HDL-NPs intravenously to SCID beige mice (C.B-*Igh-*1b/GbmsTac-*Prkdc^{scid}-Lyst^{bg}* N7) bearing flank tumor xenografts. We also tested the specificity of HDL-NP toxicity to SR-B1⁺ cells by inoculating Jurkat cells (SR-B1⁻) on the flank opposite (left) the SR-B1⁺ Ramos cells (right). Mice (*N* = 5/ group) were treated intravenously with PBS, hHDL (1 µM, 100 µL), or HDL-NP (1 µM, 100 µL) for 11 days). Mice treated with HDL-NPs had significantly smaller Ramos tumor volumes as compared to those treated with hHDL and PBS (FIG. 10A). As expected, HDL-NP treatment had no significant effect on Jurkat tumor volume (FIG. 10B). Western blotting from four representative tumor specimens obtained at necropsy on day 11 revealed that SR-B1 expression was maintained in Ramos tumors and largely absent in Jurkat tumors (FIG. 11A). Hematoxylin and eosin (H&E) staining of tissue sections obtained from Ramos and Jurkat tumors demonstrate that the presence of SR-B1, albeit minimal, observed in Western blots of Jurkat tumors is likely the result of adipocyte or other connective tissue elements present in the harvested Jurkat cell mass (FIGS. 11B-11E). Despite the reduced overall growth of the Jurkat xenografts, these data are consistent with our *in vitro* data and with SR-B1 expression measured in the tumor specimens. These data also demonstrate that HDL-NPs (100 µL) when multiply injected at a 1 µM concentration are able to outcompete natural HDLs in mouse serum, which we estimate to be at an approximate concentration equal to 20 µM.

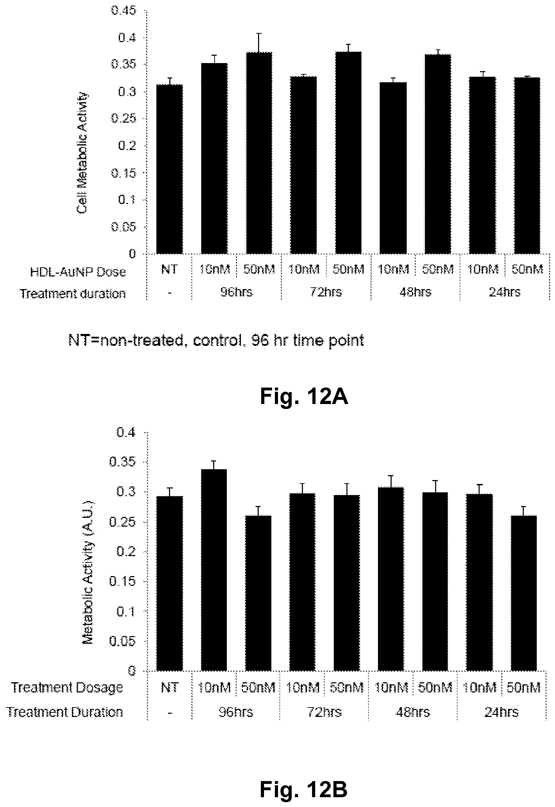

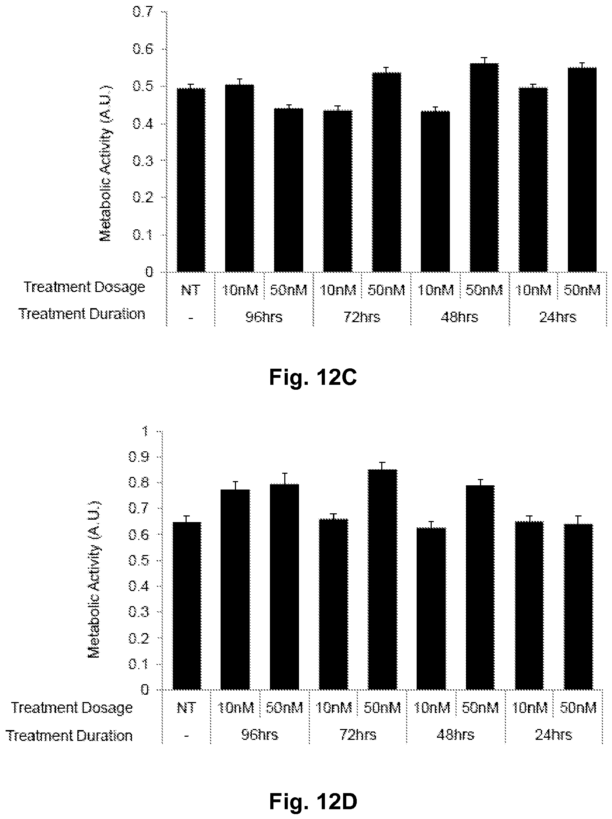

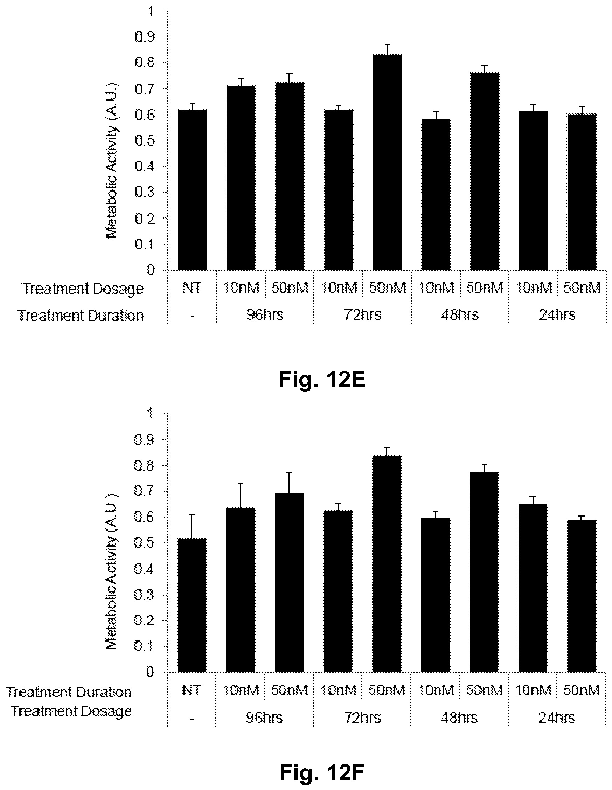

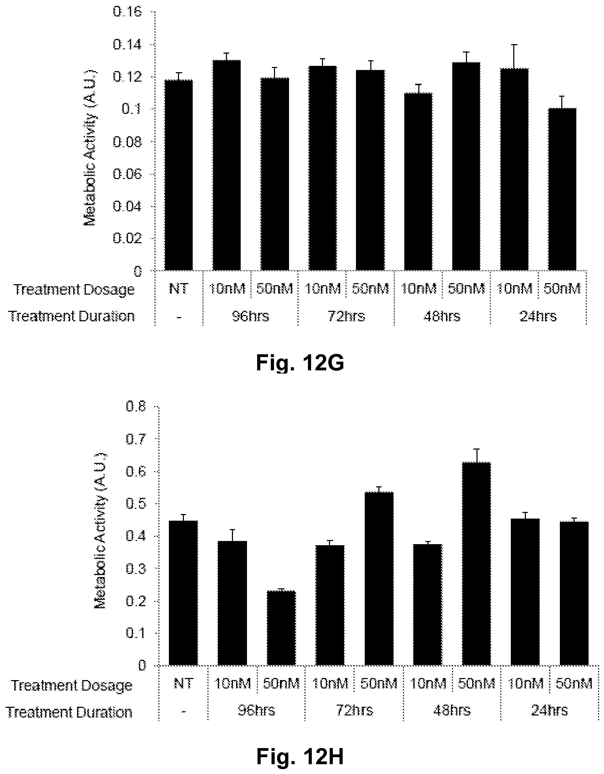

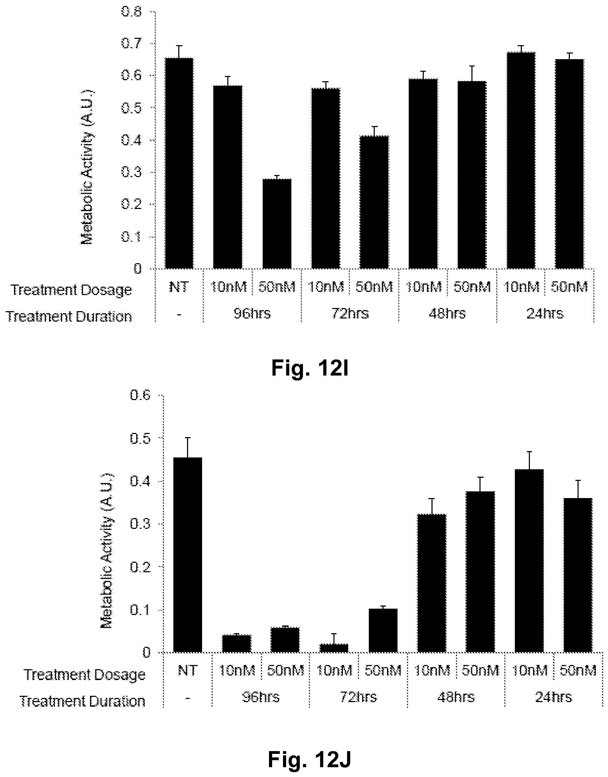

**Non lymphoma data.** Based on the data above demonstrating that nanostructures (e.g., HDL NPs) induced time- and dose-dependent apoptosis in B-cell lymphoma cell lines that express SR-B1, a screen was conducted of other cultured cells that express SR-B1 in order to assess the general applicability of nanostructures as toxic agents to cells that express SR-B1. Two different HDL NP doses (10 and 50 nM) were tested and cell viability at 24, 48, 72, and 96 hrs following treatment was measured using a colorimetric MTT cell viability assay. Comparisons were made to MTT assays conducted on untreated cells at 96 hrs. Cells that express SR-B1 and are known to interact with natural HDLs (e.g., human umbilical vein endothelial cells (HUVEC) (FIG. 12A) and liver hepatoma cell (HepG2) (FIG. 12B) were tested. In addition, a number of breast (FIGS. 12E-12G), prostate (FIGS. 12C, 12D), and melanoma cell lines (FIGS. 12H, 12I) were tested. Data demonstrate that the nanostructures reduced cell viability in the melanoma cell line, A375 (FIG. 12I). A reduction in cell viability was measured for the C8161 melanoma cell line at 96 hours (50 nM dose) (FIG. 12H). The Ramos cell line (FIG. 12J) demonstrates time- and dose-dependent apoptosis upon treatment with nanostructures, consistent with previous data. The results are shown in FIGs. 12A-12J. For all experiments, NT was the non-treated, control, at a 96 hour time point.

**Discussion.** HDL-NPs are biologically functional nanostructures that provide a new paradigm for the treatment of lymphoma. HDL-NPs induce apoptosis in B cell lymphoma cell lines *in vitro* and reduce the growth of B cell lymphoma in a xenograft model. HDL-NPs demonstrate a mechanism-of-action that may be directly dependent on the presence of the gold nanoparticle template used to control conjugate size, shape, surface chemistry, and, ultimately, control cholesterol flux at the bio-nano interface. HDL-NPs mimic spherical HDLs by targeting SR-B1 and then can differentially manipulate cellular cholesterol flux, which leads to apoptosis in B cell lymphoma cells. By contrast, hHDLs derived from human serum and Ac-LDLs are not toxic to B cell lymphoma cells.

The downstream signaling events that appear specific to B cell lymphoma cells after exposure to HDL-NPs are as yet undefined. B cell lymphoma cell lines derived from the germinal center may be most sensitive to manipulation of cellular cholesterol flux through SR-B1. The ABC-derived LY3 cells express SR-B1 and take up HDL-NPs similar to GC-derived cells, but are more resistant to apoptosis. This suggests differences in downstream signaling pathways in the ABC-derived versus the GC-derived cells, consistent with previously observed differences between ABC versus GC-derived cells. Similarly, human hepatocyte and macrophage cells, which also express SR-B1, are not susceptible to HDL-NP induced cell death. The data demonstrate the importance of cholesterol homeostasis in normal versus cancer cells, and the tight control that normal cells have over cholesterol metabolism, which is supported by our data.

B cell lymphoma cell lines derived from the germinal center may be most sensitive to manipulation of cellular cholesterol flux through SR-B1 and this provides a provocative segue to better understand the downstream mediators of this effect. Depletion of cellular cholesterol has been shown to inhibit Epstein Barr viral (EBV) infection of Burkitt's lymphoma, implicating the importance of cholesterol in EBV infection and in oncogenesis. As such, increased expression of SR-B1 by B cell lymphoma cells provides a mechanism to outcompete other tissues for cholesterol, cholesteryl esters, or viral promoters of cell growth and proliferation. Further, SR-B1 has been shown to localize in cell membrane lipid rafts and engagement of SR-B1 and manipulation of the cholesterol content and membrane fluidity, including downstream molecular pathways anchored at lipid rafts, may contribute to HDL-NP therapeutic efficacy.

Data shown in FIGS. 6 and 7 demonstrate that HDL-NPs compete with natural hHDL and Ac-LDL for binding to target cells. Importantly, the *in vivo* data demonstrate the ability of HDL-NPs to successfully compete for HDL receptors and to achieve a functionally significant reduction in tumor growth in the presence of natural, circulating HDLs. This is important because if this approach is to be utilized in patients, the competition with hHDL will be critical for success.

Embodiments described herein hold great promise for developing the next generation of novel treatments for human disease. Prevailing approaches to the synthesis of nanoparticle therapies focus on utilizing the nanoparticle as a scaffold for drug delivery, not as a functional biological entity. The therapeutic function of the former types of nanoparticles is not derived from the synthetic nanostructure itself, but from the release of active therapies contained within the nanoparticle formulation such as small molecule chemotherapies or nucleic acids. By contrast, in some embodiments described herein the nanostructures described are a functional, biomimetic nanostructure with potent therapeutic potential due to the unique ability to bind SR-B1 and/or other receptors, due to their spherical shape (among other factors), and the capacity to manipulate cholesterol flux. Due to their biomimetic nature, the nanostructures are nontoxic to normal human lymphocytes and increase the opportunities for seamless biointegration as a therapy. Finally, the nanostructures described herein may be a synthetic nanoparticle platform that can be tailored to optimize biological function such as SR-B1 binding and/or cholesterol efflux/binding properties to develop similarly potent therapies and to potentially improve upon the therapeutic properties of the biomimetic nanostructures reported here.

### Materials - Synthesis, Purification, and Characterization

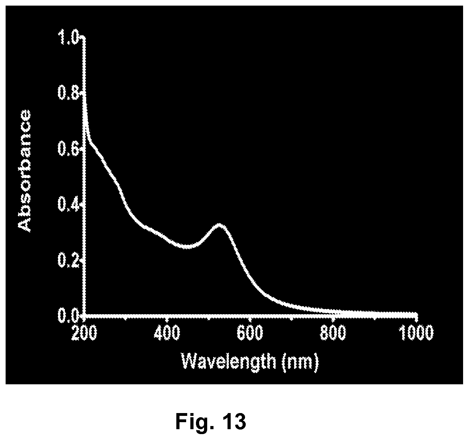

**HDL-NP Synthesis and Characterization.** Human apolipoprotein A1 (Meridian Life Sciences) was added in 5-fold molar excess to a solution of 5 nm diameter citrate-stabilized colloidal Au nanoparticles (80-100 nM, Ted Pella, Inc.). After one hour, Apo-AuNPs were diluted by 20% by adding ethanol (Sigma Aldrich). Fresh solutions of two phospholipids - 1,2-dipalmitoyl-*sn*-glycero-3-phosphoethanolamine-N-[3-(2-pyridyldithio)propionate] [PDP PE, Avanti Polar Lipids, (Dis)] and 1,2-dipalmitoyl-*sn-*glycero-3-phosphocholine (DPPC, Avanti Polar Lipids) - were prepared in ethanol at 1 mM. Each lipid was added to the solution of Apo-AuNPs in 250-fold molar excess to the AuNPs and allowed to incubate on a flat bottom shaker for 4 hours at room temperature. Next, the HDL-NPs were purified by tangential flow filtration using a Kros Flo II tangential flow filtration system (Spectrum Labs, Inc) fitted with #14 tubing and a 50 kDa MWCO modified polyethersulfone (mPES) module. For all purifications, the buffer (water) was exchanged 7 times to remove ethanol, free Apo A1, and phospholipids. The HDL-NP concentration was determined using an Agilent 8453 UV-visible spectrophotometer (5 nm colloidal gold nanoparticles, ε = 9.696 × 10⁶ M⁻¹cm⁻¹). UV-visible spectroscopy is used to measure the maximum absorbance at or near 520 nm, the wavelength corresponding to the surface plasmon resonance of 5 nm Au colloid. The HDL-NP concentration is then determined using the relationships defined by Beer's Law: *A*= *εbc*, where *A* is the absorbance, *ε* is extinction coefficient in M⁻¹ cm⁻¹, *b* is the path length of the cuvette in cm, and *c* is concentration of AuNP in M. The hydrodynamic diameter and zeta potential of the HDL-NPs (15 nM) were determined using a Malvern Zetasizer ZS (FIG. 13). The number of Apo A1 molecules per HDL-NP was determined as described in the published literature. Briefly, Apo A1 is labeled with a molecular fluorophore prior to synthesis of the HDL-NPs as described above. Then, labeled Apo A1 is liberated from a known molar quantity of HDL-NPs (see above) and the concentration is calculated based upon the fluorescent signal generated from a standard curve of labeled Apo A1.

**Table 1. Characterization of a representative sample of the hydrodynamic diameter of gold nanoparticle constructs. The hydrodynamic diameter increases from the unfunctionalized 5 nm AuNP to the HDL-NP, to which protein and lipids have been added. The λₘₐₓ values are consistent with a stable solution of AuNP.**

| | Hydrodynamic Diameter (nm) | λmax (nm) | ζ-potential (mV) | Polydispersity Index | Apo A1 per particle |
|---|---|---|---|---|---|
| 5nm AuNP | 7.5 ± 0.9 | 517 | -31 ± 11 | 0.17 ± 0.02 | 0 |
| HDL-NP | 12.8 ± 0.8 | 526 | -56 ± 3 | 0.52 ± 0.02 | 2 ± 1 |

**Calculation of Apo A1 concentration to normalize human HDL (hHDL) and HDL-NP treatments.** The molar concentration of HDL-NP is determined as discussed above and each HDL-NP has approximately three copies of Apo A1 (Table 1). Thus, Apo A1 is present in 3-fold molar excess as compared to the HDL-NP. Purchased natural HDL (Calbiochem) is derived from human serum and the total protein concentration is provided with each batch of purchased human HDL. From this value, the Apo A1 concentration is calculated based upon the assumption that Apo A1 constitutes 70% of the protein concentration of natural HDL.

### Methods

**Cell Culture.** Ramos, Jurkat, LY3, and HepG2 cells were purchased from American Type Culture Collection (ATCC). SUDHL-4 cells were from Dr. Ron Gartenhaus (University of Maryland, Baltimore, MD). Cells were cultured using standard methods. Jurkat, SUDHL-4, and Ramos cells were cultured in RPMI-1640 medium with L-glutamine, 10% fetal bovine serum (FBS) and in the presence of 1% penicillin/ streptomycin (Invitrogen). LY3 cells were grown and maintained in RPMI 1640 with L-glutamine, 15% FBS, 1% penicillin/ streptomycin, 4.5 g/L D-glucose, and 1 mM NG pyruvate. HepG2 cells were cultured in Eagle's Minimal Essential Media (EMEM) supplemented with 10% FBS. Suspended cells were cultured in T75 flasks and incubated at 37°C and 5% CO₂.

**Normal Human Lymphocyte Isolation.** Following written consent approved by the Northwestern University Institutional Review Board, peripheral blood was drawn from a healthy volunteer in Li-Heparin coated tubes. Fresh (<4 hr post donation) whole human blood was diluted 1:1 with RPMI medium. To a 50 mL tube, 10 mL of Ficoll Hystopaque and 20 mL of diluted blood were added. Samples were centrifuged (∼480*g*, 20 min, room temperature) and the upper layer of dilute medium/platelets was removed. The lymphocytes, which appeared as a milky white layer, were transferred to a new tube and diluted to 50 mL with fresh RPMI and centrifuged (∼500*g*, 6 min). The wash step was repeated twice and the final pellet was re-suspended in 5 mL of growth medium (RPMI 1640 with L-glutamine, 10% FBS and 1% penicillin/ streptomycin (Invitrogen)). If necessary, red blood cells (RBC) were removed by adding 10 mL of cold RBC lysis buffer to the lymphocyte pellet. After sitting 10 min on ice, the sample was washed twice with RPMI. Then the final pellet was re-suspended in growth medium at 1 × 10⁶ cells/mL.

**Human Hepatocyte Culture.** Primary human hepatocytes were obtained from Lonza (Walkerville, Maryland). Hepatocytes were cultured in Hepatocyte Culture Media (HCM), consisting of Hepatocyte Basal Media supplemented with Lonza's HCM SingleQuot, containing ascorbic acid, bovine serum albumin-fatty acid free, hydrocortisone, human epithelial growth factor, insulin, transferrin and Gentamincin/Amphotericin-B. Hepatocytes were seeded in HCM supplemented with 2% FBS. After 24 hours, culture media was removed and replaced with fresh HCM without FBS.

**Culture and Differentiation of CD14+ Monocytes.** Human CD14+ monocytes were obtained from Lonza. Monocytes were cultured in RPMI 1640 supplemented with 10% FBS and 1% penicillin/streptomycin. To differentiate the monocytes into macrophages, the cytokines interleukin (IL)-4 (25ng/ mL, eBioscience, San Diego, CA), IL-6 (100ng/ mL, eBioscience) and granulocyte macrophage-colony stimulating factor (GM-CSF, 100ng/ mL, eBioscience) were added to the culture media for 24 hours (3). After differentiation, culture media was replaced with fresh media without cytokines.

**MTS Assay.** For primary human hepatocytes and CD14+ monocytes, 9 × 10³ cells/ 90 µL were seeded in 96-well plates and allowed to attach, and differentiate in the case of the CD14+ monocytes, for 24 hours prior to initiation of treatment. For all other cell lines, 2.5 × 10⁴ cells/ 90 µL (24/48 hr experiments) or 1.0 × 10⁴ cells/ 90 µL (72 hr experiments) were seeded in 96-well plates. To each well, 15 µL of HDL-NP, Ac-LDL (Biomedical Technologies), hHDL (Calbiochem), or PBS was added. After incubation at 37°C for 24, 48 or 72 hours, 20 µL MTS solution (MTS - Promega, G1112) was added to each well and incubated for an additional 1 to 4 hours at 37°C. The absorbance was read at 490 nm using a microplate reader (MRX Revelation; DYNEX Technologies) and was expressed as a percentage of the control group. The control value was set to 100%. Reduction of MTS occurs in metabolically active cells, thus, the level of activity is a measure of cell viability.

**Apoptosis (Annexin V/ Propidium Iodide) Assay.** Briefly, after cell treatment and washing, 1 × 10⁵ to 1 × 10⁶ cells were labeled with Annexin V - FITC and propidium iodide (PI) reagent according to the Annexin V - FITC apoptosis detection kit instructions (Invitrogen). Cell fluorescence was read at 518 nm (FITC) and 620 nm (PI) on a Beckman Coulter FACS machine. For each analysis, 30,000 events were recorded. Results were analyzed and calculated by FCS Express V3 software and Excel. Percent apoptosis was the sum of (Annexin V-FITC⁺/PI⁻) and (Annexin V-FITC⁺/PI⁺) cells.

**Activated Caspase-3 Assay.** Briefly, 450 µL of Jurkat, Ramos and SUDHL-4 cells were plated in 24 well plates at a density of 1.1 × 10⁵ cells/mL. Over the next 72 hr, 50 µL of various treatments - including controls - were added to each well. At 72 hr, cells were collected, spun (450g, 10 min) and washed with ice-cold PBS. The cells were re-suspended in 40 µL of manufacturer provided cell lysis buffer and lysed via freeze-thaw cycles alternating between -80°C and room temperature. Next, samples were normalized for protein content by preparing 4.7 µg of protein equivalent of total cell lysates in a total volume of 20 µL [the volume deficit filled up with lysis buffer]. Using the entire volume of lysate, the assay was set up in 96-well plates and carried out per the manufacturer's protocols. The colorimetric change was measured and analyzed at the 4-hour time point. The absorbance was read at 405 nm using a microplate reader (BioTek Instruments, Synergy 2) and was expressed as a percentage of the control group.

**Western Blot.** Following cell treatments, cells were washed with PBS and centrifuged. Cell pellets were lysed with Cell Extraction Buffer (Invitrogen) supplemented with 1 mM phenylmethanesulfonylfluoride (PMSF) and Protease Inhibitor Cocktail (Sigma), and the protein concentration was measured with a colorimetric BCA Protein Assay Kit (Pierce). Total protein samples (25-50 µg) were separated on 4-20% precast polyacrylamide gels (BioRad) and transferred to polyvinylidene fluoride (PVDF) membranes. Membranes were blocked with 5% non-fat milk in TBS-T, incubated with primary antibodies followed by horseradish peroxidase (HRP)-conjugated secondary antibodies. Immunoreactive proteins were visualized using enhanced chemiluminescence. Primary antibodies: Rabbit anti-SR-Bl (Abcam ab52629), Rabbit anti-PARP (Cell signaling 9542), Rabbit anti-caspase 3 (Cell signaling 9662).

**Assay of Cellular Cholesterol Efflux.** Cells were incubated in appropriate culture media with 1µCi/mL [1,2-³H] cholesterol (Perkin Elmer Inc.) overnight to label the cellular cholesterol pool. The cells were then washed with PBS and re-suspended in appropriate, serum free culture media. Human HDL or HDL-NPs were added to the cells and incubated for 6 hours. At the end of the efflux period, the cells and culture media were collected separately and subjected to liquid scintillation counting. The percentage of cholesterol efflux was determined using the formula: counts media/ (counts cells + counts media) x 100. The background cholesterol efflux obtained in the absence of any acceptor was subtracted from the efflux values obtained with test samples.

**Assay of Cholesterol Influx.** Cells were washed with PBS and re-suspended in appropriate, serum free media with 1µCi/mL [1,2-³H] cholesterol. Human HDL or HDL-NPs were added to the cells and incubated for 6 hours. At the end of the influx period, the cells were washed with PBS. Cellular lipids were extracted with isopropanol and then subjected to liquid scintillation counting. Influx is represented as number of ³H cholesterol counts and the background cholesterol influx obtained in the absence of any acceptor (PBS) was subtracted from the influx values obtained with HDL-NPs or hHDL.

**BLT-1 Cholesterol Flux Assays.** Alterations in cholesterol flux after addition of blocker of lipid transport 1 (BLT-1) were measured as described above; however, cells were pre-treated with 10 µM BLT-1 (2-hexyl-1-cyclopentanone thiosemicarbazone, ChemBridge Corporation) for 2 hr prior to cell treatments. Following cholesterol influx or efflux, the cells were collected in fresh media for measurement of cholesterol flux as previously described. Efflux is expressed as a percentage of the control cells, which were not treated with BLT-1. Influx is expressed as a percentage of the control cells, which were not treated with BLT-1.

***In vivo* studies of HDL-NP.** *In vivo* studies were conducted with approval from the Animal Care and Use Committee (ACUC) at Northwestern University. Ramos and Jurkat cell lines were maintained in RPMI 1640 media supplemented with 10% FBS, at 5% CO₂ and 37°C. Five to six week old SCID beige mice, C.B-*Igh*-1b/GbmsTac-*Prkdc^{scid}-Lyst^{bg}* N7, were purchased from Taconic, Albany, New York. The Ramos cell line was inoculated subcutaneously on the right flank at a density of 5 × 10⁶ cells and the Jurkat cell line was inoculated on the left flank at a density of 1 × 10⁷ cells. The viability of both cells was above 90%. The animals were acclimated up to 2 days before tumor inoculation. Drug treatment was initiated after inoculated xenografts reached ∼100 mm³. The animals were randomized into three different groups: 5 mice for the control (PBS) group and 5 mice each for the treatment (hHDL and HDL-NP) groups. The control and treatment groups were intravenously injected daily (5 administrations per week) with 100 µL of PBS, 1 µM hHDL, or 1 µM HDL-NP. Side cage observation was conducted daily while tumor volume and body weight determination were measured twice weekly. At the end of the study, when Ramos xenografts reached 2,000 mm³, tumors (Ramos and Jurkat) were collected for histology. Hematoxylin and eosin (H&E) staining of the tumor samples were performed by the Mouse Histology and Phenotyping Laboratory at Northwestern University.

**Animal Tumor Immunoblot.** Briefly, 20 µg of tumor lysate was loaded in to each well of a 10% SDS-polyacrylamide (PAGE) gel. Tumor lysates were separated by SDS-PAGE, transferred to a polyvinylidine fluoride membrane, and probed as indicated. Antibodies for immunoblot analysis were obtained from the following suppliers: SR-B1 from Abcam (Cambridge, MA) and alpha-tubulin from Santa Cruz Biotechnology (Santa Cruz, CA).

**Inductively Coupled Plasma** - **Mass Spectrometry (ICP-MS).** The gold content of cell pellets in parts per billion (ppb) was determined using a standard curve and the concentration of gold nanoparticles per cell was calculated. Indium was added at 5 ppb to all samples and standard solutions as an internal standard. These values were then normalized to protein concentration.

**Transmission Electron Microscopy (TEM).** HDL-NP treated and untreated cells were pelleted and re-suspended in buffer [0.1 M sodium cacodylate (SC)], washed, and the re-suspended in fixative (2% paraformaldehyde, 2% glutaraldehyde). The cells were incubated for 30 min. at room temperature and rinsed with 0.1 M SC and placed in a secondary fixative consisting of 2% osmium tetroxide in 0.1 M SC. The cells were then rinsed and stained with distilled H₂O and 3% uranyl acetate, respectively. Once fixed, the cells were rinsed with distilled H₂O and dehydrated with ascending grades of ethanol. Propylene oxide was used as a transitional buffer, and tissues were embedded in Epon 812 and Araldite resin and cured at 60°C. The blocks were sectioned using an ultramicrotome and mounted on grids for transmission electron microscopy. TEM micrographs were obtained using a FEI Tecnai Spirit G2 at 120 kV.

**Statistics.** Data are expressed as the mean ± SD. Comparisons between two values were performed by unpaired Student's *t* test. For multiple comparisons among different groups of data, the significant differences were determined by the Bonferroni method. Significance was defined at *P* ≤ 0.05.

The indefinite articles "a" and "an," as used herein in the specification and in the claims, unless clearly indicated to the contrary, should be understood to mean "at least one."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A composition comprising a synthetic nanostructure that controls cholesterol influx and efflux in cancer cells for use in a method of treating cancer in a subject, wherein the synthetic nanostructure is a biomimic of mature, spherical high-density lipoprotein and/or is adapted to sequester cholesterol and wherein the synthetic nanostructure comprises a nanostructure core comprising an inorganic material and a shell and wherein the cancer is B-cell lymphoma, melanoma, or lymphoma, and wherein the cancer comprises cancer cells having an SR-B1 cell surface receptor.

2. The composition according to claim 1, for the use according to claim 1, wherein the cancer is non-Hodgkin lymphoma, **characterized by** B-cell lymphoma cells and **characterized by** cells having SR-B1.

3. The composition according to claim 1, for the use according to claim 1, wherein the inorganic material is a metal.

4. The composition according to claim 3, for the use according to claim 3, wherein the metal is a gold.

5. The composition according to any one of claims 1-4, for the use according to any one of claims 1-4, wherein the synthetic nanostructure core has a largest cross-sectional dimension of less than or equal to about 50 nm, or less than or equal to about 35 nm, or less than or equal to about 30 nm.

6. The composition according to any one of claims 1-5, for the use according to any one of claims 1-5, wherein the synthetic nanostructure comprises a shell comprising a lipid layer surrounding and attached to a nanostructure core.

7. The composition according to claim 6, for the use according to claim 6, wherein:
the lipid layer is a lipid bilayer
at least a portion of the lipid bilayer is covalently bound to the core,
at least a portion of the lipid bilayer is physisorbed to the core,
the lipid bilayer comprises a phospholipid, and/or
the lipid bilayer comprises 50-200 phospholipids.

8. The composition according to any one of claims 1-6, for the use according to any one of claims 1-6, wherein the shell comprises a lipoprotein structure.

9. The composition according to any one of claims 1-6, for the use according to any one of claims 1-6, wherein the shell comprises an apolipoprotein, wherein the apolipoprotein optionally is apolipoprotein A-I, apolipoprotein A-II, or apolipoprotein E.

10. The composition according to any one of claims 1-6, for the use according to any one of claims 1-6, wherein the synthetic nanostructure includes 1-6 apolipoproteins.

11. The composition according to any one of claims 1-6, for the use according to any one of claims 1-6, wherein the synthetic nanostructure comprises a shell having an inner surface and an outer surface, and a protein is associated with at least the outer surface of the shell.

12. The composition according to any one of claims 1-6, for the use according to any one of claims 1-6, wherein the synthetic nanostructure does not include a cancer drug such as a chemotherapeutic agent within the nanostructure.

13. A composition according to any one of claims 1-6, for the use according to any one of claims 1-6, wherein the synthetic nanostructure comprises a nanostructure core comprising an inorganic material and a shell comprising a lipid layer surrounding and attached to the nanostructure core, wherein the nanostructure does not include a nucleic acid.

## Patentansprüche

1. Zusammensetzung, umfassend eine synthetische Nanostruktur, welche Cholesterin-Influx und -Efflux in Krebszellen kontrolliert, zur Verwendung in einem Verfahren zur Behandlung von Krebs in einem Subjekt, wobei die synthetische Nanostruktur ein Biomimetikum eines reifen, sphärischen High-Density-Lipoprotein ist und/oder adaptiert ist, um Cholesterol zu sequestrieren und wobei die synthetische Nanostruktur einen Nanostruktur-Kern, welcher ein anorganisches Material umfasst, und eine Hülle umfasst und wobei der Krebs B-Zell-Lymphom, Melanom, oder Lymphom ist, und wobei der Krebs Krebszellen umfasst, welche einen SR-B1-Zelloberflächenrezeptor aufweisen.

2. Zusammensetzung gemäß Anspruch 1, zur Verwendung gemäß Anspruch 1, wobei der Krebs non-Hodgkin-Lymphom ist, charakterisiert durch B-Zell-Lymphom-Zellen und charakterisiert durch SR-B1 aufweisende Zellen.

3. Zusammensetzung gemäß Anspruch 1, zur Verwendung gemäß Anspruch 1, wobei das anorganische Material ein Metall ist.

4. Zusammensetzung gemäß Anspruch 3, zur Verwendung gemäß Anspruch 3, wobei das Metall ein Gold ist.

5. Zusammensetzung gemäß irgendeinem der Ansprüche 1-4, zur Verwendung gemäß irgendeinem der Ansprüche 1-4, wobei der synthetische Nanostruktur-Kern eine größte Querschnitts-Dimension von weniger als oder gleich zu ungefähr 50 nm, oder weniger als oder gleich zu ungefähr 35 nm, oder weniger als oder gleich zu ungefähr 30 nm aufweist.

6. Zusammensetzung gemäß irgendeinem der Ansprüche 1-5, zur Verwendung gemäß irgendeinem der Ansprüche 1-5, wobei der synthetische Nanostruktur-Kern eine Hülle umfasst, welche ein Lipidschicht umfasst, welche an einen Nanostruktur-Kern angeheftet ist und ihn umhüllt.

7. Zusammensetzung gemäß Anspruch 6, zur Verwendung gemäß Anspruch 6, wobei:
Die Lipidschicht eine Lipiddoppelschicht ist,
wenigstens ein Teil der Lipiddoppelschicht kovalent an den Kern gebunden ist,
wenigstens ein Teil der Lipiddoppelschicht an den Kern physisorbiert (physisorbed) ist,
die Lipiddoppelschicht ein Phospholipid umfasst,
und/oder
die Lipiddoppelschicht 50-200 Phospholipide umfasst.

8. Zusammensetzung gemäß irgendeinem der Ansprüche 1-6, zur Verwendung gemäß irgendeinem der Ansprüche 1-6, wobei die Hülle eine Lipoprotein-Struktur umfasst.

9. Zusammensetzung gemäß irgendeinem der Ansprüche 1-6, zur Verwendung gemäß irgendeinem der Ansprüche 1-6, wobei die Hülle ein Apolipoprotein umfasst, wobei das Apolipoprotein optional Apolipoprotein A-I, Apolipoprotein A-II oder Apolipoprotein E ist.

10. Zusammensetzung gemäß irgendeinem der Ansprüche 1-6, zur Verwendung gemäß irgendeinem der Ansprüche 1-6, wobei die synthetische Nanostruktur 1-6 Apolipoproteine umfasst.

11. Zusammensetzung gemäß irgendeinem der Ansprüche 1-6, zur Verwendung gemäß irgendeinem der Ansprüche 1-6, wobei die synthetische Nanostruktur eine Hülle umfasst, welche eine innere Oberfläche und eine äußere Oberfläche aufweist, und ein Protein mit mindestens der äußeren Oberfläche assoziiert ist.

12. Zusammensetzung gemäß irgendeinem der Ansprüche 1-6, zur Verwendung gemäß irgendeinem der Ansprüche 1-6, wobei die synthetische Nanostruktur kein Krebsmedikament, wie beispielweise ein chemotherapeutisches Agens, innerhalb der Nanostruktur beinhaltet.

13. Zusammensetzung gemäß irgendeinem der Ansprüche 1-6, zur Verwendung gemäß irgendeinem der Ansprüche 1-6, wobei die synthetische Nanostruktur einen Nanostruktur-Kern umfasst, welcher ein anorganisches Material und eine Hülle, welche ein Lipidschicht umfasst, welche an den Nanostruktur-Kern angeheftet ist und ihn umhüllt, umfasst, wobei die Nanostruktur keine Nukleinsäure beinhaltet.

## Revendications

1. Composition comprenant une nanostructure synthétique qui régule l'influx et l'efflux de cholestérol dans les cellules cancéreuses pour son utilisation dans un procédé de traitement du cancer chez un sujet, dans laquelle la nanostructure synthétique est un biomimétique de lipoprotéine haute densité sphérique, mature et/ou est adaptée à séquestrer le cholestérol et dans laquelle la nanostructure synthétique comprend un coeur de nanostructure comprenant un matériau inorganique et une enveloppe et dans laquelle le cancer est un lymphome à cellules B, un mélanome, ou un lymphome, et dans laquelle le cancer comprend des cellules cancéreuses ayant un récepteur de surface cellulaire SR-B1.

2. Composition selon la revendication 1, pour son utilisation selon la revendication 1, dans laquelle le cancer est un lymphome non hodgkinien, **caractérisé par** des cellules de lymphome à cellules B et **caractérisé par** des cellules ayant le SR-B1.

3. Composition selon la revendication 1, pour son utilisation selon la revendication 1, dans laquelle le matériau inorganique est un métal.

4. Composition selon la revendication 3, pour son utilisation selon la revendication 3, dans laquelle le métal est un or.

5. Composition selon l'une quelconque des revendications 1-4, pour son utilisation selon l'une quelconque des revendications 1-4, dans laquelle le coeur de nanostructure synthétique a une dimension en coupe transversale la plus grande inférieure ou égale à environ 50 nm, ou inférieure ou égale à environ 35 nm, ou inférieure ou égale à environ 30 nm.

6. Composition selon l'une quelconque des revendications 1-5, pour son utilisation selon l'une quelconque des revendications 1-5, dans laquelle la nanostructure synthétique comprend une enveloppe comprenant une couche lipidique entourant et fixée à un coeur de nanostructure.

7. Composition selon la revendication 6, pour son utilisation selon la revendication 6, dans laquelle :
la couche lipidique est une bicouche lipidique,
au moins une partie de la bicouche lipidique est liée de façon covalente au coeur,
au moins une partie de la bicouche lipidique est physisorbée au coeur,
la bicouche lipidique comprend un phospholipide, et/ou
la bicouche lipidique comprend 50 à 200 phospholipides.

8. Composition selon l'une quelconque des revendications 1-6, pour son utilisation selon l'une quelconque des revendications 1-6, dans laquelle l'enveloppe comprend une structure protéique.

9. Composition selon l'une quelconque des revendications 1-6, pour son utilisation selon l'une quelconque des revendications 1-6, dans laquelle l'enveloppe comprend une apolipoprotéine, dans laquelle l'apolipoprotéine est facultativement l'apolipoprotéine AI, l'apolipoprotéine A-II, ou l'apolipoprotéine E.

10. Composition selon l'une quelconque des revendications 1-6, pour son utilisation selon l'une quelconque des revendications 1-6, dans laquelle la nanostructure synthétique inclut 1-6 apolipoprotéines.

11. Composition selon l'une quelconque des revendications 1-6, pour son utilisation selon l'une quelconque des revendications 1-6, dans laquelle la nanostructure synthétique comprend une enveloppe ayant une surface intérieure et une surface extérieure, et une protéine est associée à au moins la surface extérieure de l'enveloppe.

12. Composition selon l'une quelconque des revendications 1-6, pour son utilisation selon l'une quelconque des revendications 1-6, dans laquelle la nanostructure synthétique n'inclut pas de médicament contre le cancer tel qu'un agent chimiothérapeutique à l'intérieur de la nanostructure.

13. Composition selon l'une quelconque des revendications 1-6, pour son utilisation selon l'une quelconque des revendications 1-6, dans laquelle la nanostructure synthétique comprend un coeur de nanostructure comprenant un matériau inorganique et une enveloppe comprenant une couche lipidique entourant le et fixée au coeur de nanostructure, dans laquelle la nanostructure n'inclut pas d'acide nucléique.
